(19) Europäisches Patentamt
European Patent Office
Office européen des brevets

(11) Veröffentlichungsnummer : **0 386 451 B1**

# (12) EUROPÄISCHE PATENTSCHRIFT

(45) Veröffentlichungstag der Patentschrift : **28.04.93 Patentblatt 93/17**

(21) Anmeldenummer : **90101943.0**

(22) Anmeldetag : **01.02.90**

(51) Int. Cl.$^5$ : **C07C 323/56,** C07C 323/62, C07C 323/22, C07C 323/19, C07C 317/44, C07C 229/60, C07C 65/26, C07C 69/76, C07C 217/48, A61K 31/10, A61K 31/135

## (54) Oxidierte Diphenylheteroalkane, ihre Herstellung und Verwendung.

(30) Priorität : **10.02.89 DE 3903988**

(43) Veröffentlichungstag der Anmeldung : **12.09.90 Patentblatt 90/37**

(45) Bekanntmachung des Hinweises auf die Patenterteilung : **28.04.93 Patentblatt 93/17**

(84) Benannte Vertragsstaaten : **AT BE CH DE DK FR GB IT LI NL SE**

(56) Entgegenhaltungen :
**EP-A- 0 133 935**
**EP-A- 0 297 995**
**FR-M- 3 710**

(73) Patentinhaber : **BASF Aktiengesellschaft**
**Carl-Bosch-Strasse 38**
**W-6700 Ludwigshafen (DE)**

(72) Erfinder : **Janssen, Bernd, Dr.**
**Leuschnerstrasse 18 a**
**W-6700 Ludwigshafen (DE)**
Erfinder : **Wüst, Hans-Heiner, Dr.**
**Unteres Bieth 10**
**W-6915 Dossenheim (DE)**

EP 0 386 451 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Es ist bekannt, daß Stilbenderivate [vgl. US 4 326 055, GB 2 164 938 und US 4 588 750], bei welchen die Polyenstruktur der Substanzen des Vitamin-A-Typs in aromatischen Ringen fixiert vorliegt, pharmakologische Wirkungen bei der topischen und systemischen Therapie von Neoplasien, Akne, Psoriasis und anderen dermatologischen Affektionen aufweisen. Die Wirkung dieser Verbindungen befriedigt jedoch nicht immer [vgl. G.L. Peck in: The Retinoids, Vol. II, 391-409, Ed.: M.B. Sporn et al., Academic Press N.Y. (1984), oder R.Marks et al., Med. J. Australia 146, 374-377 (1987), oder C.E. Orfanos et al., Drugs 34, 459-503 (1987)].

Der Erfindung lag daher die Aufgabe zugrunde, Verbindungen mit verbesserten Wirkspektren zu suchen.

Überraschend wurde nun gefunden, daß oxidierte Diphenylheteroalkane der Formel I

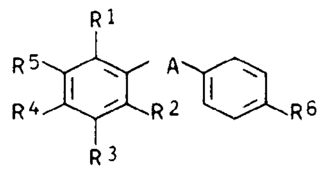

I

in der

A eine der folgenden Gruppen darstellt: $-CHOH-CH_2X-$ oder $-C(O)-CH_2X-$, wobei X mit dem rechten oder dem linken der beiden Phenylringe verbunden sein kann und für ein Sauerstoffatom, eine $-S(O)_n-$ (mit n in der Bedeutung von 0, 1 oder 2) oder eine NH-Gruppe steht,

$R^1$, $R^2$ und $R^3$ unabhängig voneinander ein Wasserstoffatom oder eine $C_1$-$C_4$-Alkylgruppe,

$R^4$ und $R^5$ unabhängig voneinander ein Wasserstoffatom, eine $C_1$-$C_5$-Alkylgruppe oder unter Bildung eines Zyklus eine gemeinsame $-C(CH_3)_2-B-C(CH_3)_2-$Gruppe (mit B in der Bedeutung von $-CH_2CH_2-$ oder $-CH(CH_3)-$) oder eine $-OC(CH_3)(Z)CH_2CH_2-$Gruppe (mit Z in der Bedeutung einer $C_1$-$C_2$-Alkylgruppe, die durch den Rest $-OR^9$ substituiert sein kann), und $R^4$ außerdem eine $-OR^9$-Gruppe bedeutet; wobei $R^4$ und $R^5$ gemeinsam einen Zyklus der genannten Art bilden, wenn $R^1$ bis $R^3$ Wasserstoff sind; und $R^4$ und $R^5$ gemeinsam einen Zyklus der genannten Art bilden oder $R^3$ und $R^5$ jeweils eine verzweigte $C_3$- oder $C_4$-Alkylgruppe darstellen, wenn $R^6$ = H, OH, SH oder $CH_3$;

$R^6$ ein Wasserstoffatom, eine Methyl- oder Nitrilgruppe, den Tetrazolylrest oder die Reste $-CH_2OR^9$, $-OR^{10}$, $-NR^{11}R^{12}$, $-CH_2NR^{11}R^{12}$, $-CH(OR^{13})_2$, $-S(O)_mR^{14}$ (mit m = 0, 1 oder 2), $-SR^{10}$, $-SO_3H$ oder $-COR^{15}$ bedeuten, worin

$R^9$ ein Wasserstoffatom, eine $C_{1-6}$-Alkyl oder eine $C_{1-6}$-Alkanoyl-Gruppe,

$R^{10}$ ein Wasserstoffatom, eine $C_{1-6}$-Alkyl-, eine $C_{1-6}$-Alkanoyl oder Benzoylgruppe oder einen $-CH_2COR^7$-Rest (mit $R^7$ in der Bedeutung von Wasserstoff, einer $C_{1-6}$-Alkyl-, einer $C_{1-6}$-Alkoxy- oder einer Hydroxy-Gruppe),

$R^{11}$, $R^{12}$ unabhängig voneinander ein Wasserstoffatom, eine $C_{1-4}$-Alkyl-, $C_{1-4}$-Alkanoyl oder eine Benzyl- oder Benzoylgruppe,

$R^{13}$ eine $C_{1-6}$-Alkoxygruppe, wobei beide Reste gegebenenfalls gemeinsam mit der CH-Gruppe ein cyclisches 5- oder 6-gliedriges Acetal bilden,

$R^{14}$ eine $C_{1-6}$-Alkylgruppe,

$R^{15}$ ein Wasserstoffatom, eine Hydroxy-, eine $C_{1-6}$-Alkyl-, eine $C_{1-6}$-Alkoxy-, Phenoxy- oder Benzyloxygruppe, den Rest $-NR^{11}R^{12}$ mit $R^{11}$ und $R^{12}$ in der Bedeutung von Wasserstoff, einer Alkyl- oder gegebenenfalls durch Hydroxy oder $C_{1-4}$-Alkoxy substituierten Benzylgruppe oder den Rest $-NR^{16}OR^{17}$ (mit $R^{16}$, $R^{17}$ = Wasserstoff oder eine $C_1$-$C_3$-Alkylgruppe) darstellen,

sowie gegebenenfalls deren physiologisch verträgliche Salze ein besseres Wirkspektrum besitzen.

Von den Verbindungen der Formel I sind diejenigen bevorzugt, in denen A eine $-XCH_2-C(O)-$ oder $-XCH_2-CHOH-$Gruppe darstellt, bei der X mit dem linken Phenylring der Formel I verknüpft ist und ein Sauerstoff- oder Schwefelatom oder die -NH-Gruppe bedeutet.

Stellen $R^1$ und/oder $R^2$ und/oder $R^3$ Alkylgruppen dar, so werden verzweigte gegenüber linearen Alkylresten bevorzugt; bilden $R^4$ und $R^5$ gemeinsam eine $-C(CH_3)_2-B-C(CH_3)_2-$Gruppe, so ist B in der Bedeutung einer $-CH_2CH_2-$Gruppe bevorzugt.

Bevorzugt sind ferner Verbindungen der Formel I, in denen $R^6$ für die Reste $-CH_2OR^9$, $-OR^{10}$, $-SR^{10}$, $-SO_2R^{14}$, $-SO_3H$ oder $COR^{15}$ steht, darunter werden solche Verbindungen besonders bevorzugt, in denen $R^9$ Wasserstoff, $R^{10}$ Wasserstoff, eine Acetylgruppe oder den Benzoylrest, der vorzugsweise mit Amino-, Acetamino-, Dimethylamino-, Hydroxy-, Methoxy- oder Methylengruppen oder Halogenatomen, insbesondere Fluor oder Chlor, substituiert ist, $R^{14}$ eine Methyl- oder Ethylgruppe, $R^{15}$ Wasserstoff, eine Hydroxylamin-, eine

Hydroxy-, eine Methyl-, Ethoxy oder Phenoxygruppe, die gegebenenfalls durch Amino-, Acetamino-, Dimethylamino-, Hydroxy- oder Methoxygruppen substituiert sein kann, oder den Rest $-NR^{11}R^{12}$ mit $R^{11}$ und/oder $R^{12}$ vorzugsweise in der Bedeutung von Wasserstoff, der Methyl- oder der Benzylgruppe, die gegebenenfalls durch Acetoxy-, Hydroxy- oder Methoxygruppen substituiert sein kann, bedeutet.

Die neuen Verbindungen der Formel I enthalten teilweise chirale Zentren und fallen im allgemeinen als Diastereomerengemische, bzw. Racemate an. Die so erhaltenen Diastereomeren lassen sich beispielsweise durch Löslichkeitsunterschiede oder durch Säulenchromatographie trennen und in reiner Form isolieren. Aus den Enantiomerenpaaren kann man nach bekannten Methoden einheitliche Enantiomere erhalten. Sowohl diese als auch ihre Gemische (Racemate) werden von der vorliegenden Erfindung umfaßt. Als therapeutische oder kosmetische Mittel kann man sowohl die einheitlichen Diastereomeren bzw. Enantiomeren wie auch deren Gemische verwenden.

Einige der erfindungsgemäßen Verbindungen weisen ein acides Wasserstoffatom auf und können daher mit Basen in üblicher Weise in ein physiologisch verträgliches, gut wasserlösliches Salz übergeführt werden. Geeignete Salze sind beispielsweise Ammonium-, Alkalimetall-, insbesondere des Natriums, Kaliums und Lithiums, oder Erdalkalimetallsalze, insbesondere des Calciums oder Magnesiums, sowie Salze mit geeigneten organischen Basen, wie mit niederen Alkylaminen, z.B. Methylamin, Ethylamin oder Cyclohexylamin, oder mit substituierten niederen Alkylaminen, insbesondere hydroxysubstituierten Alkylaminen, wie Diethanolamin, Triethanolamin oder Tris-(hydroxymethyl)-aminomethan sowie mit Piperidin oder Morpholin.

Gegebenenfalls werden die erhaltenen erfindungsgemäßen Amine der Formel (I) nach bekannter Verfahrensweise in das Säureadditionssalz einer physiologisch verträglichen Säure übergeführt. Als übliche physiologisch verträgliche organische oder anorganische Säure kommen beispielsweise in Betracht: Salzsäure, Bromwasserstoffsäure, Phosphorsäure oder Schwefelsäure und als organische Säuren beispielsweise Maleinsäure, Fumarsäure, Milchsäure, Weinsäure, Apfelsäure, Zitronensäure, Salicylsäure, Adipinsäure oder Benzoesäure. Weitere können aus "Fortschritte der Arzneimittelforschung" Band 10, Seiten 224-225, Birkhäuser Verlag, Basel und Stuttgart, 1966, entnommen werden.

Gegenstand der vorliegenden Erfindung ist weiter ein Verfahren zur Herstellung der oben angegebenen Verbindungen der Formel I, indem man

a) - falls A eine $-COCH_2X$-Gruppe bedeutet - ein Acetophenon der Formel II

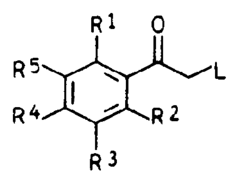

II,

in der $R^1$-$R^5$ die oben angegebene Bedeutung haben und L für eine nucleofuge Abgangsgruppe, z.B. ein Halogenatom, vorzugsweise Brom oder Chlor, oder einen reaktiven Rest $-OR^{16}$ mit $R^{16}$ in der Bedeutung einer Methylsulfonyl-, Trifluormethylsulfonyl- oder Toluolsulfonylgruppe steht, mit einem Benzolderivat der Formel III

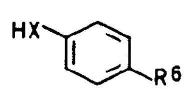

III,

worin $R^6$ die oben angegebene Bedeutung zukommt und X für ein Sauerstoff-oder Schwefelatom oder die NH-Gruppe steht, in Gegenwart einer Base umsetzt, und, falls X ein Schwefelatom bedeutet, die erhaltenen Thioether gegebenenfalls zu den korrespondierenden Sulfoxiden (X=SO) bzw. Sulfonen ($X=SO_2$) oxidiert,

b) - falls A eine $-XCH_2CO$-Gruppe bedeutet - ein Acetophenon der Formel IV

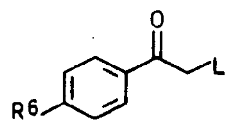

IV,

in der $R^6$ die oben angegebene Bedeutung zukommt und L für eins nucleofuge Abgangsgruppe, z.B. eine der unter (a) genannten, steht, mit Benzolderivaten der Formel V

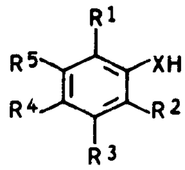

V

worin $R^1$-$R^5$ die oben angegebene Bedeutung haben und X für ein Sauerstoff- oder Schwefelatom oder die NH-Gruppe steht, in Gegenwart einer Base umsetzt, und falls X ein Schwefelatom bedeutet, die erhaltenen Thioether gegebenenfalls zu den korrespondierenden Sulfoxiden (X=SO) bzw. Sulfonen ($X=SO_2$) oxidiert,

c) - falls A eine $-CH(OH)CH_2X$-Gruppe bedeutet - entweder

c1) Oxirane der Formel VI

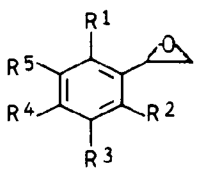

VI

in der $R^1$-$R^5$ die oben angegebenen Bedeutungen haben, mit Benzolderivaten der Formel III umsetzt und falls X ein Schwefelatom bedeutet, die erhaltenen Thioether gegebenenfalls zu den korrespondierenden Sulfoxiden (X=SO) bzw. Sulfonen ($X=SO_2$) oxidiert, oder

c2) in den Verfahren a) erhaltenen Ketonen der Formel VII

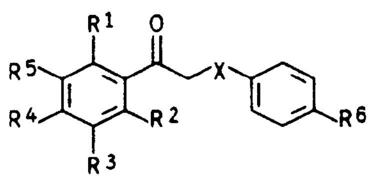

VII,

in der $R^1$-$R^6$ und X die oben angegebenen Bedeutungen haben, nach einer der bekannten Methoden die Ketogruppe reduziert,

d) - falls A eine $-XCH_2CH(OH)$-Gruppe bedeutet - entweder

d1) Oxirane der Formel VIII

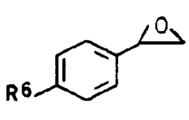

VIII,

in der $R^6$ die oben angegebene Bedeutung hat, mit Benzolderivaten der Formel V umsetzt und falls X ein Schwefelatom bedeutet, die erhaltenen Thioether gegebenenfalls zu den korrespondierenden Sulfoxiden (X=SO) bzw. Sulfonen ($X=SO_2$) oxidiert, oder

d2) in den nach Verfahren b) erhaltenen Ketonen der Formel IX

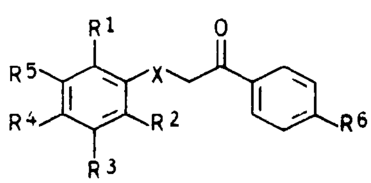

IX,

in der $R^1$-$R^6$ und X die oben angegebenen Bedeutungen haben, nach einer der bekannten Methoden die Ketogruppe reduziert.

Die Alkylierungsreaktionen a) und b) erfolgen in an sich bekannter Weise, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C, wobei man bevorzugt äquimolare Mengen der Reaktanten II und III bzw. IV und V oder eine Komponente gegebenenfalls im Überschuß von bis zu 10 Mol% einsetzt. Zu den bevorzugten Lösungs- oder Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, die gegebenenfalls auch als säurebindende Mittel bei der Reaktion verwendet werden können, sind beispielsweise Alkalicarbonate wie Natrium- oder Kaliumcarbonat oder Natrium- und Kaliumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyltriethylammoniumchlorid oder -bromid oder Kronenether wie 12-Krone-4, 15-Krone-5, 18-Krone-6, Di-benzo-18-Krone-6 oder Dicyclohexano-18-Krone-6 in Frage.

Die Umsetzung wird im allgemeinen drucklos oder unter Druck, kontinuierlich oder diskontinuierlich durchgeführt.

Bei der Durchführung der erfindungsgemäßen Verfahren c1) und d1) setzt man vorzugsweise auf 1 Mol Oxiran VI bzw. VIII 1 bis 2 Mol der HX-Komponente III bzw. V und gegebenenfalls 1 bis 2 Mol Base ein. Als Verdünnungsmittel kommen unter den Reaktionsbedingungen inerte organische Lösungsmittel in Frage. Hierzu gehören vorzugsweise Alkohole, wie Ethanol, Methoxyethanol oder Propanol; Ketone, wie Aceton oder 2-Butanon; Nitrile, wie Acetonitril; Ester, wie Essigester; Ether, wie Dioxan oder Tetrahydrofuran; aromatische Kohlenwasserstoffe, wie Benzol oder Toluol; Amide, wie Dimethylformamid oder N-Methylpyrrolidon; Sulfoxide, wie Dimethylsulfoxid, ferner Sulfolan oder entsprechende Gemische. Als Basen kommen für die erfindungsgemäßen Umsetzungen gemäß Verfahren c1) und d1) alle üblicherweise verwendbaren anorganischen und organischen Basen in Betracht. Hierzu gehören vorzugsweise Alkalicarbonate, wie Natrium- und Kaliumcarbonat; Alkalihydroxide, wie Natriumhydroxid; Alkalialkoholate, wie Natrium- und Kalium-methylat und -ethylat; Alkalihydride, wie Natriumhydrid; sowie niedere tertiäre Alkylamine, Cycloalkylamine und Aralkylamine, wie insbesondere Triethylamin.

Die Reaktionstemperaturen können in einem größeren Bereich variiert werden. Im allgemeinen arbeitet man bei Temperaturen zwischen 0°C und 200°C, vorzugsweise zwischen 60°C und 150°C.

Man kann die Verfahren c1) und d1) aber auch in Gegenwart von basischem Aluminiumoxid bei Raumtemperatur durchführen.

Die Durchführung der Verfahren c2) und d2) erfolgt nach allgemein üblichen Methoden zur Reduktion von Ketonderivaten. Dabei setzt man auf 1 Mol der Carbonylverbindungen VII bzw. IX 1 bis 3, vorzugsweise 1 bis 1,5 Äquivalente des Reduktionsmittels ein.

Als Reduktionsmittel kommen Metallhydride wie Diisobutylaluminiumhydrid, Natrium-, Lithium- und Kaliumhydrid, -borhydrid oder -cyanoborhydrid, aber auch Lithiumaluminiumhydride der allgemeinen Formel

$$Li\,Al\,(H)_m\,(OR)_n$$

mit m = 1 bis 4 und n = 4- m, wobei R gleich oder verschieden sein kann und allgemein für Alkylreste, z.B. Methyl, Ethyl, Propyl, iso-Propyl, n-Butyl, iso-Butyl, tert.-Butyl oder Cyclohexyl steht, oder Wasserstoff, gegebenenfalls in Gegenwart eines geeigneten Katalysators, z.B. Rhodium oder Ruthenium, in Frage. Besonders bevorzugt ist Natriumborhydrid.

Die Reaktion wird im allgemeinen bei Temperaturen zwischen -20°C und +120°C, bevorzugt bei -5°C bis +50°C, besonders bevorzugt bei 0°C - +30°C drucklos oder unter Druck, kontinuierlich oder diskontinuierlich durchgeführt.

Die nach den erfindungsgemäßen Verfahren a) - d) erhaltenen Thioether (X=S) werden, falls dies erwünscht ist, oxidativ in die korrespondierenden Sulfoxide (X=SO) oder Sulfone (X=$SO_2$) übergeführt. Die Darstellung der Sulfoxide erfolgt gegebenenfalls in Gegenwart eines Lösungs- oder Verdünnungsmittels und gegebenenfalls unter Zusatz eines Katalysators, indem man 1,0 Äquivalente des Thioethers mit 1,0 bis 1,1 Äquivalenten des Oxidationsmittels bei Temperaturen von -30 bis 120°C zu dem entsprechenden Sulfoxid umsetzt.

Die Darstellung der entsprechenden Sulfone erfolgt durch Verwendung von 2,0 bis 3,0 Äquivalenten an Oxidationsmittel. Zu den bevorzugten Lösungs-oder Verdünnungsmitteln gehören niedere Alkylcarbonsäuren wie Ameisensäure, Essigsäure und Propionsäure, Alkohole wie Methanol, Ethanol oder Isopropanol, Kohlenwasserstoffe wie Hexan, Cyclohexan oder Heptan, Aromaten wie Benzol, Toluol oder Xylol, Ether wie Methyl-tert.-butylether, Diisopropylether oder Diphenylether, Ketone wie Aceton oder Methylethylketon, halogenierte Kohlenwasserstoffe wie Dichlormethan, Chloroform, Tetrachlorkohlenstoff, Dichlorethan oder Chlorbenzol, aber auch Nitrile wie Acetonitril und Propionitril oder Amide wie Dimethylformamid, Dimethylacetamid oder Pyrrolidon sowie Wasser; es sind aber auch Gemische derselben geeignet.

Als Oxidationsmittel kommen in Frage Peroxyverbindungen wie Wasserstoffperoxid, tert. Butylhydroperoxid, Peressigsäure, Perbenzoesäure, mono-Perphthalsäure oder halogenierte Perbenzoesäuren wie m-Chlorperbenzoesäure. Es sind aber auch andere Oxidationsmittel wie Kaliumpermanganat, Kaliumdichromat, Natriumperjodat oder Perjodsäure sowie Salpetersäure oder nitrose Gase wie Stickstoffdioxid verwendbar (vgl. beispielsweise "Methoden der Organischen Chemie" Hrsg. Eugen Müller, Bd. IX, S. 207 ff und S. 223 ff, Thieme Verlag, Stuttgart 1955, sowie Reid, "Organic Compounds of Bivalent Sulfur", Bd. 2, S. 64 ff, Chem. Publ. Comp. New York, 1960).

Als Katalysatoren kommen gegebenenfalls in Betracht Mineralsäuren wie Chlorwasserstoffsäure oder Schwefelsäure, aber auch Alkalihydroxide wie Natrium- oder Kaliumhydroxid sowie Alkalicarbonate wie Natrium- oder Kaliumcarbonat.

Im Falle, daß die Reaktion in einem zweiphasigen System abläuft, können Phasentransferkatalysatoren, z.B. quarternäre Ammoniumverbindungen wie Tetrabutylammoniumsalze, zur Reaktionsbeschleunigung verwendet werden.

Die Ausgangsverbindungen der Formel II (mit L = Wasserstoff (z.B. DE-OS 3 602 473, DE-OS 3 434 942, DE-OS 3 434 944), Chlor oder Brom) sind teilweise bekannt oder werden erhalten nach üblichen Methoden zur Darstellung von Acetophenonen, beispielsweise durch Friedel-Crafts Acylierung mit Essigsäure, bzw. Chlor- oder Bromessigsäure, deren Halogeniden oder Anhydriden (H.O. House: "Modern Synthetic Reactions", 2nd Ed., W.A. Benjamin Inc. Menlo Park, CA; 1972, dort S. 797 ff und dort zitierte Literatur) oder durch Oxidation der entsprechenden Ethylbenzole (H.O. House, l.c., S. 288 f und dort angegebene Literatur). So erhaltene Verbindungen der Formel II (mit L = Wasserstoff) werden anschließend in an sich bekannter Weise halogeniert (H.O. House, l.c., S. 459-478 und dort angegebene Literatur).

Die Darstellung der reaktiven Ester der Formel II, worin L den Rest $-OR^{16}$ bedeutet, erfolgt aus den entsprechenden ω-Hydroxy-acetophenon II (mit L = OH) nach allgemein bekannten Methoden (Houben-Weyl-Müller, "Methoden der organischen Chemie", Georg Thieme Verlag, Stuttgart, 1955, Band IX, Seiten 388, 663, 671). Solche Ester sind beispielsweise Methansulfonsäureester, Trifluormethansulfonsäureester, Nonafluorbutansulfonsäureester, 4-Methylbenzolsulfonsäureester, 4-Brombenzolsulfonsäureester, 4-Nitrobenzolsulfonsäureester oder Benzolsulfonsäureester.

Die Ausgangsverbindungen der Formel III sind bekannt, bzw. sie wurden nach den üblichen Methoden zur Darstellung para-substituierter Aniline, Phenole und Thiophenole erhalten.

Die Ausgangsverbindungen der Formel IV sind zum Teil bekannt oder lassen sich nach allgemein üblichen Verfahren - wie für die Acetophenone der Formel II oben beschrieben - herstellen.

Die Ausgangsverbindungen der Formel V sind teilweise bekannt oder können - im Falle, daß X ein Sauerstoffatom bedeutet - nach den allgemein üblichen Methoden zur Herstellung substituierter Phenolderivate erhalten werden ("Methoden der Organischen Chemie", Hrsg. Eugen Müller, Bd. VI/1c, S, 4 ff, S. 313 ff, S. 925 ff, Thieme Verlag, Stuttgart, 1976); oder lassen sich - im Falle, daß X ein Schwefelatom bedeutet - nach den für die Darstellung von Thiophenolen üblichen Verfahren erhalten (K.-D. Gundermann und K. Hümke in "Methoden der Organischen Chemie", Bd. E11, S. 32 ff, Thieme Verlag, Stuttgart, 1985 und dort zitierte Literatur). Sie lassen sich bevorzugt herstellen durch Reduktion der entsprechenden Sulfonsäurederivate zum Beispiel mit Metallhydriden oder auch aus den entsprechenden Phenolen der Formel II, die zu Thiocarbaminsäureestern umgesetzt werden (Newman u. Karnes, J. Org., Chem. 31, 3980 (1966)); oder sie werden - im Falle, daß X eine NH-Gruppe bedeutet - in an sich bekannter Weise hergestellt, beispielsweise durch Reduktion der entsprechenden Nitroverbindungen ("Methoden der Organischen Chemie", Hrsg. E. Müller, Bd. XI/1, S. 394, Thieme Verlag, Stuttgart, 1957).

Die Ausgangsverbindungen der Formel VI sind überwiegend noch nicht bekannt, sie können jedoch in allgemein bekannter Art und Weise erhalten werden, indem man die entsprechenden Benzaldehyd-Derivate der Formel X,

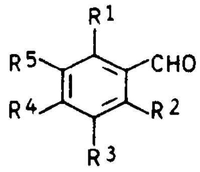

X,

in der $R^1$-$R^5$ die oben angegebene Bedeutung haben, entweder

α) mit Dimethyloxosulfonium-methylid der Formel (XI),

$$(CH_3)_2S^{\oplus}OCH^{\ominus}_2 \qquad XI$$

in an sich bekannter Weise in Gegenwart eines Verdünnungsmittels, beispielsweise Dimethylsulfoxid, bei Temperaturen zwischen 20°C und 80°C umsetzt (vgl. hierzu die Angaben in J. Am. Chem. Soc. 87, 1363-1364 (1965)) oder

β) mit Trimethylsulfonium-methylsulfat der Formel (XII),

$$[(CH_3)_3S^{\oplus}]\ CH_3SO_4^{\ominus} \qquad XII$$

in an sich bekannter Weise in Gegenwart eines inerten organischen Lösungsmittels, wie Acetonitril, und in Gegenwart einer Base, wie Natriummethylat, bei Temperaturen zwischen 0°C bis 60°C, vorzugsweise bei Raumtemperatur, umsetzt (vgl. auch die Angaben in Heterocycles 8, 397 (1977)).

Die Ausgangsaldehyde der Formel X sind zum Teil bekannt oder werden erhalten nach üblichen Methoden zur Darstellung von Benzaldehyden, beispielsweise durch Aromatenformylierung nach Vilsmeier (vgl. De. Meheas, Bull. Soc. Chem. Fr. 1989-1999 (1962) und dort zitierte Literatur) oder durch Reduktion der entsprechenden Benzoylhalogenide (vgl. Fuson in: Patai, "The Chemistry of the Carbonyl Group", Vol. 1, S. 211-232, Interscience Pupl., N.Y. 1966 oder Wheeler in: Patai, "The Chemistry of Acyl Halides", S. 231-251, Interscience Pupl. N.Y, 1972) oder der entsprechenden Benzonitrile (vgl. J. March: "Advanced Organic Chemistry, 2nd Ed., McGraw-Hill Kogakusha LTD, Tokyo, 1977, S. 835-836 und dort zitierte Literatur).

Die Ausgangsverbindungen der Formel VIII sind zum Teil bekannt; sie werden vorzugsweise hergestellt aus den korrespondierenden Benzaldehyden der Formel XIII,

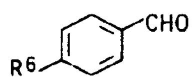

XIII

in der $R^6$ die oben angegebene Bedeutung zukommt, analog den für die Darstellung von Oxiranen des Typs VI oben angegebenen Verfahren. Die Ausgangsaldehyde der Formel XIII sind überwiegend bekannt oder werden in an sich bekannter Art und Weise, wie für Aldehyde der Formel X oben angegeben, erhalten.

Die nach den oben genannten Verfahren a-d hergestellten Substanzen lassen sich anschließend wie folgt weiter abwandeln:

Die Benzoesäureester der Formel I ($R^6$ = Carboalkoxy) werden, falls erwünscht, durch Esterverseifung in die freien Carbonsäuren übergeführt. Umgekehrt läßt sich natürlich die freie Säure in bekannter Weise verestern.

Zweckmäßigerweise wird die Verseifung/Veresterung in Gegenwart eines Verdünnungs- oder Lösungsmittels, beispielsweise eines Dialkylglykolethers oder cyclischen Ethers, wie 1,2-Dimethoxyethan, Tetrahydrofuran oder Dioxan, eines niederen aliphatischen Ketons, wie Aceton, Methylethylketon oder Methylisobutylketon, oder in einem niederen aliphatischen Alkohol, wie Methanol, Ethanol, Propanol oder Isopropanol, oder in Dimethylsulfoxid, gegebenenfalls in Gegenwart von Wasser bzw. in Mischungen der genannten Lösungsmittel mit Wasser durchgeführt.

Bevorzugte Lösungsmittel sind wäßrige Mischungen wie Ethanol, Methanol und Dimethylsulfoxid, wobei die Umsetzung beim Siedepunkt des Reaktionsgemisches durchgeführt wird.

Die Verseifung geschieht bevorzugt in Gegenwart von Alkali, wie Alkalimetallhydroxiden, Alkalimetallcarbonaten und -hydrogencarbonaten, insbesondere des Natriums und Kaliums, organischen tertiären Basen, wie Pyridin oder niederen Trialkylaminen, wie Trimethyl- oder Triethylamin in Gemischen mit Wasser. Dabei wird die verwendete Base im Verhältnis zum Ester in stöchiometrischer Menge oder in geringem Überschuß eingesetzt. Vorzugsweise wird Natrium- oder Kaliumhydroxid verwendet.

Die Veresterung geschieht vorteilhaft, indem man die Carbonsäure zunächst in ihr Salz überführt und dieses mit einem entsprechenden Alkylhalogenid, vorzugsweise einem Alkylbromid oder -iodid, behandelt. Als Deprotonierungsmittel für die Herstellung der Salze in situ eignen sich insbesondere die Carbonate, Hydroxide und Hydride der Alkalimetalle.

Zweckmäßigerweise verwendet man aprotische polare Lösungsmittel wie Aceton, Dimethylformamid, Dimethylsulfoxid und insbesondere Methylethylketon, wobei die Umsetzung beim Siedepunkt des Reaktionsgemisches durchgeführt wird.

Die erfindungsgemäßen Amide der allgemeinen Formel I können in an sich bekannter Weise hergestellt werden, indem man die Benzoesäuren I ($R^6$ = COOH) zunächst in carbonylaktivere Derivate überführt, z.B. in die Säurehalogenide, -azide, -imidazolide oder -anhydride, die O-Acyl-N,N'-dicyclohexylisoharnstoffe oder p-Nitrophenylester, und diese mit Aminen $HNR^{11}R^{12}$ behandelt. In Fällen besonders reaktiver Amine, vor allem Ammoniak, ist die direkte Amidolyse von Estern (mit $R^6$ = $-COR^{15}$ und $R^{15}$ in der Bedeutung von Alkoxy) bevorzugt.

Die erfindungsgemäßen Hydroxamsäure-Derivate der allgemeinen Formel I werden hergestellt, indem man Benzoesäuren der Formel I ($R^6$ - $CO_2H$) in die entsprechenden carbonylaktiveren Derivate - wie oben angegeben - überführt und diese anschließend mit Hydroxylamin oder seinen Derivaten, $HNR^{16}OR^{16}$, in einem inerten organischen Lösungsmittel, gegebenenfalls unter Zusatz einer organischen oder anorganischen Base als Protenenfänger, vorzugsweise bei Raumtemperatur oder auch bei erhöhter Temperatur drucklos oder unter Druck zur Reaktion bringt.

Im Falle von Hydroxylamin als Reaktant setzt man dieses bevorzugt als Salz, z.B. als Hydrochlorid, ein und führt die Reaktion unter Verwendung von 2 Äquivalenten Base, z.B. Triethylamin oder Kaliumcarbonat, in Dimethylformamid durch.

Eine Carbonsäure, ein Carbonsäureester oder ein Carbonsäureamid der Formel I ($R^6$ = $C(O)R^{15}$) kann in an sich bekannter Weise zu den entsprechenden Alkoholen bzw. Aminen reduziert werden. Vorteilhaft wird die Reduktion mit Hilfe eines Metallhydrids oder Alkalimetallhydrids in Gegenwart eines geeigneten Lösungsmittels durchgeführt. Als Metallhydride werden vorzugsweise komplexe Metallhydride wie Lithiumaluminiumhydrid oder Diisobutylaluminiumhydrid eingesetzt. Als Lösungsmittel werden beim Arbeiten mit Lithiumaluminiumhydrid Ether eingesetzt, wie Diethylether, Dioxan oder Tetrahydrofuran. Führt man die Reduktion mit Diisobutyl aluminiumhydrid oder einem Alkoxynatriumaluminiumhydrid durch, so ist die Verwendung von Kohlenwasserstoffen wie Hexan oder Toluol bevorzugt.

So erhaltene Amine oder Alkohole können in an sich bekannter Weise mit einem Alkanoylhalogenid oder -anhydrid oder einem Aroylhalogenid oder -anhydrid zweckmäßigerweise in einem inerten Verdünnungs- oder Lösungsmittel, z.B. einem niederen aliphatischen Keton wie Aceton, Methylethylketon oder Methylisobutyleton, einem Dialkylformamid wie Dimethylformamid oder Diethylformamid oder mit einem überschüssigem Acylierungsmittel als Verdünnungs- oder Lösungsmittel in die erfindungsgemäßen Amide und Ester der Formel (I) übergeführt werden. Bevorzugt werden die Umsetzungen in Gegenwart einer Base als säurebindendem Mittel in einem zwischen -20°C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Acylierungsmittel in katalytischer Menge oder in stöchiometrischer Menge bzw. in geringem Überschuß verwendet werden.

Ein unter die Formel I fallender Alkohol ($R^6$ = $CH_2OR^9$, $R^9$ = H) kann mit Alkylhalogeniden $R^9$-J, $R^9$-Br oder $R^9$-Cl in Gegenwart von Alkalimetallhydriden, vorzugsweise Natriumhydrid oder in Gegenwart von Alkyllithium-Verbindungen, vorzugsweise n-Butyllithium in einem organischen Lösungsmittel wie Tetrahydrofuran, Dioxan, 1,2-Dimethoxyethan, Methyl-tert.-butylether oder bei Verwendung von Natriumhydrid auch in Dimethylformamid in einem zwischen -10°C und 40°C liegenden Temperaturbereich zum entsprechenden Ether umgesetzt werden.

Ein unter die Formel I fallender Alkohol kann mit geeigneten Oxidationsmitteln, vorzugsweise Mangan(IV)-oxid, gegebenenfalls auf einem anorganischen Trägermaterial wie Silicagel oder Aluminiumoxid zum entsprechenden Aldehyd oxidiert werden. Vorteilhaft arbeitet man in einem inerten organischen Lösungsmittel, beispielsweise einem Kohlenwasserstoff wie Hexan oder in einem Ether, beispielsweise Tetrahydrofuran, oder in Mischungen der genannten Lösungs- und Verdünnungsmittel im Temperaturbereich zwischen -10°C und 30°C. Die benötigte Reaktionszeit ist im wesentlichen von der Oxidationsaktivität des eingesetzten Mangan(IV)-oxids abhängig.

Einen Aldehyd I ($R^6$ = -CHO) kann man auch durch Reduktion des entsprechenden Nitrils mit Diisobutylaluminiumhydrid in einem Lösungsmittel, vorzugsweise in Toluol, Hexan, Tetrahydrofuran oder Mischungen dieser Lösungsmittel, in einem Temperaturbereich zwischen -40°C und Raumtemperatur erhalten.

Die unter die Formel I fallenden Aldehyde und Ketone werden auch dadurch erhalten, daß man ihre Acetale hydrolysiert, üblicherweise in Gegenwart einer Säure als Katalysator, vorzugsweise verdünnter Salz- oder Schwefelsäure, bei 20°C oder bei bis zum Siedepunkt des Reaktionsgemisches erhöhter Temperatur. Zweckmäßigerweise arbeitet man in mit Wasser mischbaren Lösungsmitteln wie Aceton, Dioxan, Tetrahydrofuran, vorzugsweise in kurzkettigen Alkoholen wie Methanol und Ethanol.

Ein Nitril der Formel I ($R^6$ = -CN) kann in an sich bekannter Weise unter Säure- oder vorteilhafter Basenkatalyse zur entsprechenden Carbonsäure verseift werden. Als Basen bevorzugt sind Alkalimetallhydroxide, besonders Kaliumhydroxid, das im Überschuß eingesetzt wird. Als Lösungsmittel werden in der Regel mit Wasser mischbare Alkohole wie Methanol, Ethanol, Isopropanol oder n-Butanol eingesetzt. Die Umsetzung wird üblicherweise beim Siedepunkt des Reaktionsgemisches durchgeführt.

Aus den Nitrilen I ($R^6$ = -CN) können durch Addition eines Azides, z.B. eines Alkalimetallazids, vorzugsweise Natriumazid, in Gegenwart von Aluminiumchlorid oder Ammoniumchlorid die entsprechenden Tetrazole erhalten werden. Als Lösungsmittel verwendet man bevorzugt cyclische Ether, wie Dioxan oder Tetrahydrofuran sowie insbesondere Dimethylformamid oder deren Mischungen, wobei die Umsetzung im allgemeinen bei einer Temperatur von 60-100°C abläuft.

Die acylierten Phenole der allgemeinen Formel I ($R^6$ = -$OR^{10}$ mit $R^{10}$ = $C_1$-$C_6$-Alkanoyl) werden, falls dies erwünscht ist, in die freien Phenole und ihre physiologisch verträglichen Salze durch Esterverseifung übergeführt. Zweckmäßigerweise erfolgt die Verseifung in Gegenwart eines Verdünnungsmittels, beispielsweise eines mit Wasser mischbaren Ethers, wie 1,2-Dimethoxyethan oder Tetrahydrofuran, oder eines niederen aliphatischen Alkohols, wie Methanol, Ethanol, Propanol, Isopropanol oder Butanol, in Gegenwart von Wasser bzw. in Mischungen der genannten Lösungsmittel mit Wasser. Bevorzugte Lösungsmittel sind wäßrige Mischungen von Ethanol oder Methanol, wobei die Umsetzung zwischen 20°C und dem Siedepunkt des Reaktionsgemisches durchgeführt wird. Die Verseifung geschieht bevorzugt in Gegenwart von Hydroxiden oder Carbonaten der Alkali- und Erdalkalimetalle, insbesondere des Natriums und Kaliums.

Ein Phenol der Formel I kann in an sich bekannter Weise mit einem Alkanoylhalogenid oder -anhydrid oder einem Aroylhalogenid oder -anhydrid in einem inerten Verdünnungs- oder Lösungsmittel, z.B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungsmittel oder Lösungsmittel in die erfindungsgemäßen Ester übergeführt werden. Bevorzugt werden die Umsetzungen in Gegenwart einer Base als säurebindendem Mittel in einem zwischen -20°C und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Alkylierungsmittel in katalytischer Menge oder in stöchiometrischer Menge bzw. in geringem Überschuß verwendet werden.

Die Veretherung der Phenole der Formel I zu Arylethern der Formel I geschieht vorteilhaft, indem man das Phenol zunächst in sein Salz überführt und dieses mit einem entsprechenden Alkylhalogenid oder -sulfat, vorzugsweise einem Alkylchlorid, -bromid oder -jodid, behandelt. Als Deprotonierungsmittel für die Herstellung der Phenolate in situ eignen sich insbesondere die Carbonate, Hydroxide und Hydride der Alkalimetalle. Zweckmäßigerweise verwendet man aprotische polare Lösungsmittel wie Aceton, Dimethylformamid, Dimethylsulfoxid oder Methylethylketon, wobei die Umsetzung zwischen 20°C und dem Siedepunkt des Reaktionsgemisches durchgeführt wird.

Die acylierten Thiophenole der allgemeinen Formel I ($R^6$ = -SC(O)$R^{14}$ mit $R^{14}$ = $C_1$-$C_6$-Acyl) werden, falls dies erwünscht ist, in freie Thiophenole und ihre physiologisch verträglichen Salze durch Esterverseifung übergeführt. Zweckmäßigerweise erfolgt die Verseifung in Gegenwart eines Verdünnungsmittels, beispielsweise eines mit Wasser mischbaren Ethers, wie 1,2-Dimethoxyethan oder Tetrahydrofuran, oder eines niederen aliphatischen Alkohols, wie Methanol, Ethanol, Propanol, Isopropanol oder Butanol, in Gegenwart von Wasser bzw. Mischungen der genannten Lösungsmittel mit Wasser. Bevorzugte Lösungsmittel sind wäßrige Mischungen von Ethanol oder Methanol, wobei die Umsetzung zwischen 20°C und dem Siedepunkt des Reaktionsgemisches durchgeführt wird. Die Verseifung geschieht bevorzugt in Gegenwart von Hydroxiden oder Carbonaten der Alkali- und Erdalkalimetalle, insbesondere des Natriums und Kaliums.

Ein Thiophenol der Formel I kann in an sich bekannter Weise mit einem Alkanoylhalogenid oder -anhydrid oder einem Aroylhalogenid oder -anhydrid zweckmäßigerweise in einem inerten Verdünnungs- oder Lösungsmittel, z.B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungsmittel oder Lösungsmittel in die erfindungsgemäßen Thioester übergeführt werden. Bevorzugt werden die Umsetzungen in Gegenwart einer Base als säurebindendem Mittel in einem zwischen -20°C und dem Siedepunkt des Reaktionsgemisches liegenden Temperaturbereich vorgenommen. Geeignete Basen sind Alkalimetallcarbonate, -hydrogencarbonate, -hydroxide oder alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Alkylierungsmittel in katalytischer Menge oder in stöchiometrischer Menge bzw. in geringem Überschuß verwendet werden.

Die Veretherung der Thiophenole der Formel I zu Arylthioethern der Formel I geschieht vorteilhaft, indem man das Thiophenol zunächst in sein Salz überführt und dieses mit einem entsprechenden Alkylhalogenid, oder -sulfat, vorzugsweise einem Alkylchlorid, -bromid oder -jodid, behandelt. Als Deprotonierungsmittel für die Herstellung der Thiophenolate in situ eignen sich insbesondere die Carbonate, Hydroxide und Hydride der Alkalimetalle. Zweckmäßigerweise verwendet man aprotische polare Lösungsmittel wie Aceton, Dimethylformamid, Dimethylsulfoxid oder Methylethylketon, wobei die Umsetzung zwischen 20°C und dem Siedepunkt des Reaktionsgemisches durchgeführt wird.

Die erfindungsgemäßen Thioether der Formel I ($R^6$ = $-S-C_1-C_6$-Alkyl) werden, falls dies erwünscht ist, in die entsprechenden Sulfoxide bzw. Sulfone übergeführt. Die Oxidation zu Sulfoxiden erfolgt vorteilhaft, indem man den Thioether in alkoholischer Lösung mit äquimolaren Mengen oder einem bis zu 10-prozentigen Überschuß von Perjodsäure oder einem Alkalisalz derselben, vorzugsweise dem Natriumsalz, bei Temperaturen von 0 bis 30°C zur Reaktion bringt. Als Löslichkeitsvermittler sind beispielsweise Wasser, Dimethylsulfoxid oder Amide wie Dimethylformamid, aber auch Ketone wie Aceton geeignet. Die Oxidation zu Sulfonen erfolgt vorteilhaft, indem man 2,0 bis 3,0 Äquivalente des Oxidationsmittels bei Temperaturen von -30 bis 1200°C, vorzugsweise bei -10 bis 60°C auf den entsprechenden Thioether einwirken läßt. Als Oxidationsmittel eignen sich Wasserstoffperoxid und besonders Peroxicarbonsäuren, worunter m-Chlor-peroxibenzoesäure bevorzugt wird. Als Lösungsmittel werden bei der Verwendung von Wasserstoffperoxid vorzugsweise Essigsäure oder Acetonitril, bei der Verwendung von Peroxicarbonsäuren aprotische Lösungsmittel wie Methylenchlorid oder Toluol eingesetzt.

Die Thiophenole der Formel I ($R^6$ = SH) können, falls dies erwünscht ist, in die korrespondierenden Sulfonsäuren übergeführt werden, indem man auf das jeweilige Thiophenol - vorteilhaft in Essigsäure - bei Temperaturen von 10°C bis zum Siedepunkt der Reaktionslösung die 2- bis 5-fache molare Menge Wasserstoffperoxid einwirken läßt.

Aus den erfindungsgemäßen N-acylierten Aminen der Formel I ($R^6$ = $-NH-C_1-C_4$-Alkanoyl oder -Benzoyl) erhält man durch saure oder alkalische Hydrolyse die korresponierenden Anilinderivate ($R^6$ = $NH_2$). Zweckmäßigerweise wird die Hydrolyse in Gegenwart eines Lösungs- oder Verdünnungsmittels, beispielsweise eines Dialkylglykolethers oder cyclischen Ethers, wie 1,2-Dimethoxyethan, Tetrahydrofuran oder Dioxan, eines niederen aliphatischen Ketons, wie Aceton, Methylethylketon, Methylisobutylketon, oder eines niederen aliphatischen Alkohols, wie Methanol, Ethanol, Propanol oder Isopropanol, bzw. in Mischungen der genannten Lösungsmittel mit Wasser durchgeführt.

Bevorzugte Lösungsmittel sind wäßrige Mischungen von Ethanol und Methanol, wobei die Umsetzung beim Siedepunkt des Reaktionsgemisches durchgeführt wird.

Die alkalische Verseifung geschieht in Gegenwart von Alkali, wie Alkalimetallhydroxiden, Alkalimetallcarbonaten und -hydrogencarbonaten, insbesondere des Natriums und Kaliums, organischen tertiären Basen, wie Pyridin oder niederen Trialkylaminen, wie Trimethyl- oder Triethylamin in Gemischen mit Wasser. Dabei wird die verwendete Base im Verhältnis zum Ester in stöchiometrischer Menge oder in geringem Überschuß eingesetzt. Vorzugsweise wird Natrium- oder Kaliumhydroxid verwendet.

Die saure Verseifung erfolgt in Gegenwart von Mineralsäuren, wie Chlorwasserstoffsäure, Schwefelsäure oder Phosphorsäure, oder von organischen Säuren, wie Benzolsulfonsäure oder Toluolsulfonsäure. Dabei wird die verwendete Säure im Verhältnis zum Ester in stöchiometrischer Menge oder in geringem Überschuß eingesetzt. Vorzugsweise wird Chlorwasserstoffsäure verwendet.

So erhaltene Aniline ($R^6$ = $NH_2$) können in an sich bekannter Weise mit einem Alkanoylhalogenid oder -anhydrid oder einem Aroylhalogenid oder -anhydrid oder einem Alkyl- oder Benzylhalogenid zweckmäßigerweise in einem inerten Verdünnungs-oder Lösungsmittel, z.B. einem niederen aliphatischen Keton wie Aceton, Methylethylketon oder Methylisobutylketon, einem Dialkylformamid wie Dimethylformamid oder Diethylformamid oder mit überschüssigem Acylierungsmittel als Verdünnungs- oder Lösungsmittel in die erfindungsgemäßen Amine und Amide der Formel (I) ($R^6$ = $NR^{11}R^{12}$) übergeführt werden. Bevorzugt werden die Umsetzungen in Gegenwart einer Base als säurebindendem Mittel in einem zwischen -20°C und dem Siedepunkt des Reaktionsgemisches liegenden Temperaturbereich vorgenommen. Geeignete Basen sind z.B. Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Acylierungs- oder Alkylierungsmittel in katalytischer Menge oder in stöchiometrischer Menge bzw. in geringem Überschuß verwendet werden.

Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und inneren Organe sowie bei der topischen und systemischen Therapie von Akne, Psoriasis und anderer mit pathologisch veränderter Verhornung einhergehenden dermatologischen Erkrankungen, insbesondere Ichthyosis, Dariersche Krankheit, Flechte, Leukoplakie, aber auch gegen Vitiligo, Ekzeme, Warzen, Lichtschäden (vorzeitige Alterung) der Haut, ferner gegen trockene Augen und andere Corneopathien sowie zur Behandlung von rheumatischen Erkrankungen, insbesondere solchen entzündlicher oder degenerativer Art, die Gelenke, Muskeln, Sehnen und andere Teile des Bewegungsapparates befallen, verwendet werden. Bevorzugte Indikationsgebiete sind: Die Therapie von

dermatologischen Erkrankungen; die Therapie von durch Sonnenlichteinwirkung bedingten Hautschädigungen; die Therapie von iatrogen, z.B. durch Corticosteroide induzierte Atrophie, bedingten Hautschädigungen und die prophylaktische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertrachealgewebe in vitro die nach Vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. 36, 964-972 (1972) oder aus Nature 250, 64-66 (1974) und Nature 253, 47-50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035-1041 (1978), Experimental Cell Research 117, 15-22 (1978) und Proc. Natl. Acad. Sci. USA 77, 2937-2940 (1980) entnommen werden.

Die antiarthritische Wirkung der erfindungsgemäßen Verbindungen kann in üblicher Weise im Tierexperiment im Adjuvans-Arthritis- oder Streptokokkenzellwandinduzierten-Arthritis-Modell bestimmt werden. Die dermatologische Aktivität, beispielsweise für die Behandlung von Akne, kann u.a. durch die komedolytische Aktivität und die Fähigkeit nachgewiesen werden, die Anzahl der Zysten im Modell der Rhino-Maus zu reduzieren.

Diese Methode ist von L.H. Kligman et al. in The Journal of Investigative Dermatology 73, 354-358 (1978) beschrieben. Als weiteres Maß für die dermatologische Aktivität kann die Reduktion der Talgdrüsen und die damit einhergehende verminderte Talgproduktion am Seitenorgan des Hamsters dienen. Diese Methodik ist von E.C. Gomez in J. Am. Dermatol. 6, 746-750 (1982) beschrieben.

Ferner kann die durch die erfindungsgemäßen Verbindungen erzielbare Reversion von Hautschäden, welche durch UV-Licht ausgelöst wurden, in Tiermodellen bestimmt werden. Diese Methodik ist von L.H. Kligman et al. beschrieben in Connect. Tissue Res. 12, 139-150 (1984) und im Journal of the American Academy of Dermatology 15, 779-785 (1986).

Dementsprechend sind ein weiterer Gegenstand der Erfindung kosmetische und therapeutische Mittel zur topischen und systemischen Anwendung, die eine Verbindung der Formel (I) als Wirkstoff neben üblichen Trägerstoffen oder Verdünnungsmitteln enthalten.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen oder kosmetischen Mittel können die erfindungsgemäß zu verwendenden Verbindungen bei lokaler Anwendung in 0,001 bis 1 %iger Konzentration, bevorzugt in 0,001 bis 0,1 %iger Konzentration und bei systemischer Anwendung als therapeutische Mittel vorzugsweise in einer Einzeldosis von 0,1 bis 250 mg enthalten und täglich in einer oder mehreren Dosierungen je nach Art und Schwere der Erkrankungen verabreicht werden.

Die Arzneimittel und Kosmetika der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprenmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Zweckmäßige übliche Bestandteile von kosmetischen und pharmazeutischen Zubereitungen für die topische Anwendung sind beispielsweise:
anionische, kationische sowie nichtionische Emulgatoren und Emulsionsstabilisatoren, die gleichzeitig Konsistenzgeber oder Gelbildner sein können, wie Polyvinylpyrrolidon, Fettalkohole, Glycerinmonostearat, Polyacrylsäuren, Cellulosederivate und Ethylenoxid-Propylenoxid-Blockpolymere,
feste oder flüssige Ölkomponenten bzw. Fettstoffe mineralischer, pflanzlicher oder tierischer Herkunft, synthetische Esteröle, wie Triglyceridester und Isopropylmyristat,
hydrophile Komponenten, wie Glycerin, Polyethylenglykol und Propylenglykol.

Weiterhin können als Inhaltsstoffe von Kosmetika genannt werden beispielsweise Lichtschutzmittel, Bräunungsmittel, Konservierungsmittel, Antioxidantien, Pigmente, Farbstoffe, ätherische Öle und Parfümöle, Vitamine, Pflanzenextrakte, Collagen usw. Diese Stoffe können beispielsweise dem CTFA, Cosmetic Ingredient Dictionary, 3. Auflage, Washington 1982, entnommen werden.

Herstellung der Ausgangsverbindungen

Beispiel A

5,6,7,8-Tetrahydro-5,5,8,8-tetramethyl-2-naphthalenyloxiran

34 g (0,3 mol) Kalium-tert.-butanolat wurden in 300 ml trockenem Dimethylsulfoxid bei 20-25°C mit 66 g (0,3 mol) Trimethylsulfonoxiumjodid versetzt, nach 30 min. wurde eine Lösung aus 65 g (0,3 mol) 2-Formyl-5,6,7,8-tetrahydro-5,5,8,8-tetramethylnaphthalin in 100 ml trockenem Dimethylsulfoxid und 200 ml trockenem Tetrahydrofuran zugetropft. Nach beendeter Reaktion wurde auf Eiswasser gegossen, mit Ether extrahiert, die organische Phase mit Wasser gewaschen, über Magnesiumsulfat getrocknet und das Lösungsmittel im Vakuum abgedampft. Der Rückstand lieferte 60 g der Titelverbindung als gelbliches Öl.

$^1$H-NMR ($CDCl_3$): $\delta$ = 3,8 (t,1H)

Beispiel B

Phenyloxiran-4-carbonsäuremethylester

Analog zu Beispiel A wurden aus 49,0 g (0,3 mol) 4-Formylbenzoesäuremethylester 37 g der Titelverbindung als gelbliches Öl hergestellt.

$^1$H-NMR ($CDCl_3$): $\delta$ = 3,2 (dd,1H)

Beispiel C

ω-Brom-acetophenon-4-carbonsäuremethylester

13,5 g (82 mmol) Acetophenon-4-carbonsäure wurden in 100 ml Dimethylformamid mit 13,8 g (100 mmol) wasserfreiem Kaliumcarbonat und nach 30 min. tropfenweise mit 12,6 g (100 mmol) Dimethylsulfat versetzt. Nach 1 Std. Rühren bei 60°C wurde die erkaltete Reaktionslösung auf Wasser gegossen, der Niederschlag wurde abgesaugt, mit Wasser gewaschen und getrocknet. Man erhielt 11,6 g Acetophenon-4-carbonsäuremethylester. 1,8 g (10 mmol) des so hergestellten Esters wurden in 20 ml Eisessig bei Raumtemp. mit 0,6 ml (10 mmol) Brom tropfenweise versetzt. Nach beendeter Reaktion wurde auf Wasser gegossen; der Niederschlag wurde abfiltriert, mit Wasser gewaschen und lieferte nach Trocknung 2,3 g der Titelverbindung. Die daraus durch alkalische Verseifung erhaltene Carbonsäure schmilzt bei 190 bis 192°C.

Beispiel D

ω-Chlor-5,6,7,8-tetrahydro-5,5,8,8-tetramethyl-2-acetonaphthon

In eine Suspension aus 36 g (0,27 mol) wasserfreiem Aluminiumchlorid in 135 ml trockenem Methylenchlorid und 14 ml (0,18 mol) Chloressigsäurechlorid wurden 34 g (0,18 mol) 1,2,3,4-Tetrahydro-1,1,4,4-tetramethylnaphthalin, gelöst in 100 ml trockenem Methylenchlorid, bei 0-5°C eingetropft. Der Ansatz rührte über Nacht bei Raumtemp. und wurde anschließend auf Eiswasser gegossen. Die organische Phase wurde abgetrennt, mit Wasser und gesättigter Kochsalzlösung gewaschen, über Magnesiumsulfat getrocknet und im Vakuum eingedampft. Der Rückstand lieferte 43 g der Titelverbindung als gelbliches Öl.

$^1$H-NMR ($CDCl_3$): $\delta$ = 1,3 (12H), 1,66 (4H), 4,7 (2H)

Beispiel E

2,2,5,7,8-Pentamethyl-chroman-6-ol

In eine Suspension von 248 g (1,86 mol) wasserfreiem Aluminiumchlorid in 850 ml trockenem Dichlormethan wurden bei 5°C 257 g (1,69 mol) 2,3,6-Trimethyl-hydrochinon eingetragen. Nach 30 min. Rühren wurden bei 5-10°C 160 g (1,86 mol) 3-Methyl-2-buten-1-ol zugetropft. Der Ansatz rührte über Nacht bei Raumtemperatur, wurde anschließend auf Eiswasser gegossen, der Niederschlag wurde abfiltriert, das Filtrat mit Dichlormethan extrahiert; die organische Phase wurde mit Wasser neutral gewaschen, über Magnesiumsulfat getrocknet und eingedampft. Destillation des Rückstandes lieferte bei 140°C und 0,4 mbar eine Hauptfraktion von 153,3 g der Titelverbindung, die aus Heptan umkristallisiert einen Schmp. von 85-88°C aufwies.

Aus dem zuvor angefallenen Niederschlag konnten weitere 10,3 g des Produktes isoliert werden.

Beispiel F

3,5-Di-tert.-butyl-thiophenol

Zu 26,5 g (0,6 mol) Natriumhydrid (ca. 60 %ige Suspension in Mineralöl), welches zuvor mit Pentan entölt und in 200 ml trockenem Dimethylformamid suspendiert worden war, wurde eine Lösung aus 103,2 g (0,5 mol) 3,5-Di-tert.-butyl-phenol in 150 ml Dimethylformamid unter Rühren bei Raumtemp. zugetropft. Nach beendeter Wasserstoffentwicklung ließ man 67,5 g (0,55 mol) N,N-Dimethylthiocarbamidsäurechlorid in 100 ml Dimethylformamid zutropfen, anschließend wurde 1 Stunde lang bei 70°C gerührt, die Reaktionslösung wurde in 500 ml kalte Kalilauge (5 %ig) eingerührt, der Niederschlag abfiltriert, gewaschen und getrocknet. 84 g des so erhaltenen Produktes wurden unter Stickstoff 30 min auf 320°C erhitzt. Die erstarrte Schmelze lieferte nach Umkristallisation aus Diisopropylether 42 g eines farblosen Feststoffes (Schmp.: 103-105°C), der in 200 ml Ether gelöst bei Raumtemp. zu einer Suspension aus 7 g (0,18 mol) Lithiumaluminiumhydrid in 800 ml Ether getropft wurde. Nach beendeter Reaktion wurden dem Gemisch 100 ml 2 N Salzsäure zugesetzt, die Etherphase abgetrennt, mit Wasser gewaschen, über Magnesiumsulfat getrocknet und im Vakuum eingedampft. Der Rückstand lieferte 28,8 g der Titelverbindung, Schmp. 47-49°C.

Beispiel G

5,6,7,8-Tetrahydro-5,5,8,8-tetramethyl-2-naphthol

250,0 g (2,65 mol) Phenol wurden mit 414,5 g (2,27 mol) 2,5-Dichlor-2,5-dimethylhexan in 500 ml Petrolether bei Raumtemperatur unter Rühren mit 27,5 g (0,2 mol) wasserfreiem Aluminiumchlorid spatelweise versetzt. Nach 48 Stunden wurde das Reaktionsgemisch auf Eiswasser gegossen, mit Ether extrahiert und die organische Phase mit Wasser neutral gewaschen, über Natriumsulfat getrocknet und im Vakuum eingedampft. Der Rückstand ergab nach zweifachem Umkristallisieren aus Methanol 148,7 g der Titelverbindung, Schmp. 219-220°C.

Beispiel H

5,6,7,8-Tetrahydro-5,5,8,8-tetramethyl-2-naphthylamin

Zu einer Lösung 65,8 g (0,35 mol) 1,2,3,4-Tetrahydro-1,1,4,4-tetramethylnaphthalin in 154 ml Eisessig und 257 ml Essigsäureanhydrid wurde im Eis-Kochsalzbad ein unter Kühlen bereitetes Gemisch aus 77,2 ml Eisessig und 20,8 ml Salpetersäure (98 %ig) binnen 2 Stunden zugetropft. Nach beendeter Zugabe wurde das Reaktionsgemisch auf Raumtemperatur erwärmt und über Nacht gerührt. Die Lösung wurde danach auf Wasser gegossen, der Niederschlag abgesaugt, mit Wasser nachgewaschen und getrocknet. Man erhielt 79,7 g rohes 2-Nitro-5,6,7,8-tetrahydro-5,5,8,8-tetramethyl-naphthalin vom Schmp. 46-50°C.

25 g dieses Rohproduktes wurde in einem Gemisch aus 65 ml Dioxan, 65 ml Methanol und 5 ml Wasser über 0,3 g Palladium auf Aktivkohle (10 %) während 48 Stunden bei 100°C und 200 bar Wasserstoff im Autoklaven hydriert. Nach beendeter Reaktion wurde vom Katalysator abfiltriert und die Lösung eingedampft. Umkristallisieren des Rückstandes aus n-Heptan lieferte 17,6 g der Titelverbindung, Schmp. 63-65 °C.

Beispiel J

5,6,7,8-Tetrahydro-5,5,8,8-tetramethyl-2-thionaphthol

94,0 g (0,5 mol) 1,2,3,4-Tetrahydro-1,1,4,4-tetramethylnaphthalin wurden binnen 30 min bei 20 °C in 100 ml Chlorsulfonsäure eingerührt. Die Reaktionslösung blieb 1 Stunde bei 60 °C, wurde nach dem Abkühlen auf Raumtemperatur auf 1,5 l Eis gegossen und mit Ether extrahiert. Die organische Phase wurde mit wäßriger

Kochsalzlösung und Wasser neutral gewaschen, über Magnesiumsulfat getrocknet und im Vakuum eingedampft. Umkristallisieren des Rückstandes aus Methanol ergab 55,0 g 5,6,7,8-Tetrahydro-5,5,8,8-tetramethyl-naphthalin-2-sulfonsäurechlorid, Schmp. 71-74 °C.

57,5 g (0,2 mol) des so hergestellten Sulfonsäurechlorids wurden in 150 ml trockenem Tetrahydrofuran binnen 2 Stunden bei Raumtemperatur zu einer Suspension aus 15,4 g (0,4 mol) Lithiumaluminiumhydrid in 150 ml trockenem Tetrahydrofuran getropft. Es wurde 1 Stunde bei Raumtemperatur nachgerührt, die Reaktionslösung tropfenweise mit 25 ml Wasser, danach mit 50 ml gesättigter Weinsäurelösung versetzt und wenige Minuten zum Sieden erhitzt. Nach dem Abkühlen wurde die Lösung mit wasserfreiem Magnesiumsulfat bis zum Aufklaren versetzt und vom Niederschlag abgesaugt.

Eindampfen des Filtrats erbrachte 33,0 g der Titelverbindung, die als Harz anfiel ($R_F$ = 0,4, Heptan/Essigester = 7:3).

Herstellung der erfindungsgemäßen Verbindungen

Beispiel 1

1-(4-Methoxycarbonyl-phenyl)-2-(5,6,7,8-tetrahydro-5,5,8,8-tetramethyl-2-naphthylthio)-ethanol

2,2 g (10 mmol) 5,6,7,8-Tetrahydro-5,5,8,8-tetramethyl-2-thionaphthol (aus Beispiel J) und 1,78 g (10 mmol) Phenyloxiran-4-carbonsäuremethylester (aus Beispiel B) wurden mit 15 g basischem Aluminiumoxid (Woelm) in 50 ml Toluol bei Raumtemperatur gerührt. Nach beendeter Reaktion wurde die Lösung filtriert und im Vakuum eingedampft. Der Rückstand lieferte nach Chromatographie (Heptan/Essigester = 95:5) an 30 g Kieselgel 0,4 g der Titelverbindung als farbloses Öl.

$^1H$-NMR ($CDCl_3$): $\delta$ = 3,22 (d,2H); 3,84 (s,3H); 4,79 (m,1H)

Beispiel 2

1-(4-Methoxycarbonylphenyl)-2-(5,6,7,8-tetrahydro-5,5,8,8-tetramethyl-2-naphthyloxy)-ethanol

2 g (10 mmol) 5,6,7,8-Tetrahydro-5,5,8,8-tetramethyl-2-napthol (aus Beispiel G) und 1,78 g (10 mmol) Phenyloxiran-4-carbonsäuremethylester (aus Beispiel B) wurden mit 7 g (50 mmol) Kaliumcarbonat und 10 g Aluminiumoxid in 50 ml Toluol unter Rückfluß erhitzt. Nach beendeter Reaktion wurde die Lösung filtriert, das Filtrat eingedampft und der Rückstand an 14 g Kieselgel chromatographiert (Heptan/Essigester = 95:5).

Man erhielt 0,3 g der Titelverbindung vom Schmp. 92-96°C

Beispiel 3

1-(5,6,7,8-Tetrahydro-5,5,8,8-tetramethyl-2-naphthalenyl)-2-(4-carboxyphenylthio)-ethanon

Zu einer Lösung aus 15,0 g (0,1 mol) 4-Mercaptobenzoesäure und 22,0 g (0,22 mol) Triethylamin in 200 ml Dimethylformamid wurden bei 0-5°C 26,5 g (0,1 mol) w-Chlor-5,6,7,8-tetrahydro-5,5,8,8-tetramethyl-2-acetonaphthon (aus Beispiel D) in 100 ml Dimethylformamid zugetropft. Nach 2 Std. Rühren wurde die Reaktionslösung auf Wasser gegossen, mit verdünnter Salzsäure auf pH 4 gestellt, der Niederschlag abgesaugt, mit Wasser und Methanol gewaschen und getrocknet. Umkristallisieren aus Methanol lieferte 16,2 g der Titelverbindung, Schmp. 175-190°C. Aus der Mutterlauge konnten weitere 2,2 g reines Produkt erhalten werden.

Beispiel 4

5,6,7,8-Tetrahydro-5,5,8,8-tetramethyl-2-acetonaphthonyl-(4-carboxyphenyl)-sulfon

5,0 g (13 mmol) 1-(5,6,7,8-Tetrahydro-5,5,8,8-tetramethyl-2-naphthalenyl)-2-(4-carboxyphenylthio)-ethanon (aus Beispiel 3) wurden in 100 ml Eisessig bei 50°C tropfenweise mit 5 ml (30 mmol) Wasserstoffperoxid (30 %ige wässrige Lösung) versetzt und 1 Std. bei 75°C gehalten. Die erkaltete Reaktionslösung wurde auf verdünnte wässrige Kochsalzlösung gegossen, der Niederschlag abgesaugt und nach Trocknung an 35 g Kieselgel chromatographiert (Methylenchlorid/Methanol = 98:2) und lieferte 2,0 g der Titelverbindung, die aus Heptan/Essigester kristallisierte, Schmp. 156-160°C.

Beispiel 5

1-(5,6,7,8-Tetrahydro-5,5,8,8-tetramethyl-2-naphthalenyl)-2-(4-ethoxycarbonyl-phenoxy)-ethanon

14,3 g (55 mmol) w-Chlor-5,6,7,8-tetrahydro-5,5,8,8-tetramethyl-2-acetonaphthon (aus Beispiel D) wurden in 220 ml Dimethylformamid spatelweise mit 6,6 g (55 mmol) Kalium-tert.-butanolat versetzt. Bei

Raumtemp. wurde eine Lösung aus 9,1 g (55 mmol) 4-Hydroxybenzoesäureethylester in 330 ml Dimethylformamid zugetropft und das Reaktionsgemisch nach vollständiger Umsetzung auf Wasser gegossen und mit Ether extrahiert; die Extrakte wurden mit Wasser gewaschen, über Magnesiumsulfat getrocknet und eingedampft. Der ölige Rückstand lieferte nach chromatographischer Reinigung (Heptan/Essigester = 85:15) und Kristallisation aus Methanol 16,4 g der Titelverbindung, Schmp. 105-107°C.

Beispiel 6

1-(5,6,7,8-Tetrahydro-5,5,8,8-tetramethyl-2-naphthalenyl)-2-(4-carboxy-phenoxy)-ethanon

6 g (15 mmol) 1-(5,6,7,8-Tetrahydro-5,5,8,8-tetramethyl-2-naphthalenyl)-2-(4-ethoxycarbonyl-phenoxy)-ethanon (aus Beispiel 5) wurden mit 5 g Kaliumhydroxid in 90 ml Ethanol/Wasser/Dimethylsulfoxid (5:3:1) 5 min. bei 80°C gerührt, auf Eiswasser gegossen, die alkalische Lösung mit Ether extrahierte und der Extrakt verworfen, anschließend mit konz. Salzsäure auf pH 3 gestellt und die saure Lösung mit Essigester extrahiert. Dieser Extrakt wurde mit Wasser gewaschen, über Magnesiumsulfat getrocknet und eingedampft. Umkristallisieren des Rückstandes aus Ethanol lieferte 2,8 g der Titelverbindung, Schm. 168-170°C.

Beispiel 7

1-(5,6,7,8-Tetrahydro-5,5,8,8-tetramethyl-2-naphthalenyl)-2-(4-carboxy-phenoxy)-ethanol

2,1 g (5 mmol) 1-(5,6,7,8-Tetrahydro-5,5,8,8-tetramethyl-2-naphthalenyl-2-(4-ethoxycarbonyl-phenoxy)-ethanon (aus Beispiel 5) wurden in 100 ml Tetrahydrofuran/Methanol (2:1) mit 0,4 g (10 mmol) Natriumborhydrid 30 min bei Raumtemp. gerührt; die Reaktionslösung wurde auf gesättigte Kochsalzlösung gegossen und mit Essigester extrahiert; die Extrakte lieferten nach Waschen mit Wasser, Trocknen über Magnesiumsulfat und Eindampfen einen öligen Rückstand, der wie in Beispiel 6 beschrieben der alkalischen Hydrolyse unterworfen wurde und nach entsprechender Aufarbeitung 1,4 g der Titelverbindung erbrachte; Schmp. (Cyclohexan): 160-162°C.

Beispiel 8

1-(5,6,7,8-Tetrahydro-5,5,8,8-tetramethyl-2-naphthalenyl)-2-(4-carboxyphenylamino)-ethanon

115 g (0,5 mol) 5,6,7,8-Tetrahydro-5,5,8,8-tetramethyl-2-acetonaphthon wurden in 100 ml Eisessig unter Zusatz von 4 Tropfen Bromwasserstoffsäure in Eisessig (30 %ige Lösung) bei 30°C mit 25,5 mol (0,5 mol) Brom tropfenweise versetzt und nach beendeter Reaktion in 1,5 l Eiswasser eingetragen. Extraktion mit Methylenchlorid, Waschen der Extrakte mit Wasser und verdünnter Natriumhydrogencarbonatlösung sowie Trocknen über Natriumsulfat lieferte nach Abdampfen des Lösungsmittels 90 g eines öligen Rohproduktes, wovon 48 g in 80 ml Dimethylformamid aufgenommen und unter Eisbadkühlung zu einer Suspension aus 25 g (0,18 mol) 4-Aminobenzoesäure und 40 g (0,4 mol) Triethylamin in 70 ml Dimethylformamid getropft wurden. Nach 3 Std. Rühren bei Raumtemperatur wurden 200 ml Wasser zugetropft. Der braune Niederschlag wurde abgetrennt und lieferte nach Umkristallisieren aus Methanol/Tetrahydrofuran 16,7 g der Titelverbindung, Schmp. 168-169°C.

Beispiel 9

1-(4-Methoxycarbonyl-phenyl)-2-(5,6,7,8-tetrahydro-5,5,8,8-tetra-methyl-2-naphthylthio)-ethanon

2,0 g (9 mmol) 5,6,7,8-Tetrahydro-5,5,8,8-tetramethyl-2-thionaphthol wurden in 30 ml Dimethylformamid spatelweise mit 1,2 g (10 mmol) Kalium-tert.-butanolat und anschließend bei Raumtemp. tropfenweise mit 2,3 g (9 mmol) w-Brom-4-methoxycarbonyl-acetophenon (aus Beispiel C) versetzt. Nach 30 min. wurde die Reaktionslösung in Wasser eingetragen und mit Ether extrahiert; die Extrakte lieferten nach Waschen mit Wasser, Trocknen über Magnesiumsulfat und Abdampfen des Lösungsmittels einen Rückstand, aus dem nach Digerieren mit Heptan 1,3 g der Titelverbindung als Öl anfielen.

$^1$H-NMR ($CDCl_3$): $\delta$ = 3,95 (s,3H); 4,2 (s,2H)

Analog wurden die in der nachfolgenden Tabelle angegebenen Beispiele hergestellt:

Tabelle

| Beispiel Nr. | $R^1$ | $R^2$ | $R^3$ | $R^4$ | $R^5$ | A | $R^6$ | Schmp. (°C) |
|---|---|---|---|---|---|---|---|---|
| 10 | H | H | H | $-C(CH_3)_2CH_2CH_2C(CH_3)_2-$ | | $-CHOH-CH_2-S-$ | $-CO_2H$ | 158-160 |
| 11 | H | H | H | $-C(CH_3)_2CH_2CH_2C(CH_3)_2-$ | | $-CHOH-CH_2-NH-$ | $-CO_2H$ | 96-104 |
| 12 | H | H | H | $-C(CH_3)_2CH_2CH_2C(CH_3)_2-$ | | $-S-CH_2-CHOH-$ | $-OC(CH_3)_3$ | |
| 13 | H | H | $C(CH_3)_3$ | H | $C(CH_3)_3$ | $-O-CH_2-CHOH-$ | $-OC(CH_3)_3$ | |
| 14 | H | H | $C(CH_3)_3$ | H | $C(CH_3)_3$ | $-O-CH_2-CHOH-$ | $-OH$ | |
| 15 | H | H | $C(CH_3)_3$ | H | $C(CH_3)_3$ | $-O-CH_2-CHOH-$ | $-OC(O)CH_3$ | |
| 16 | H | H | H | $-C(CH_3)_2CH(CH_3)C(CH_3)_2-$ | | $-NH-CH_2-CHOH-$ | $-OC(CH_3)_3$ | 139-142 |
| 17 | H | H | H | $-C(CH_3)_2CH_2CH_2C(CH_3)_2-$ | | $-O-CH_2-CHOH-$ | $-SCH_3$ | |
| 18 | H | H | $C(CH_3)_3$ | H | $C(CH_3)_3$ | $-O-CH_2-CHOH-$ | $-SCH_3$ | 70- 73 |
| 19 | $CH_3$ | $CH_3$ | $CH_3$ | $-OC(CH_3)_2CH_2CH_2-$ | | $-O-CH_2-CHOH-$ | $-SCH_3$ | |
| 20 | H | H | $C(CH_3)_3$ | H | $C(CH_3)_3$ | $-O-CH_2-CHOH-$ | $-S(O)_2C_2H_5$ | |
| 21 | H | H | $C(CH_3)_3$ | H | $C(CH_3)_3$ | $-O-CH_2-CHOH-$ | $-CN$ | 119-122 |
| 22 | H | H | $C(CH_3)_3$ | H | $C(CH_3)_3$ | $-O-CH_2-CHOH-$ | $-CH_2NH_2$ | 148-150 |
| 23 | H | H | H | $-C(CH_3)_2CH_2CH_2C(CH_3)_2$ | | $-S-CH_2-CHOH-$ | $-NHC(O)CH_3$ | |
| 24 | $CH_3$ | $CH_3$ | $CH_3$ | $-OC(CH_3)_2CH_2CH_2-$ | | $-O-CH_2-COOH-$ | $-NHC(O)CH_3$ | |
| 25 | H | H | $C(CH_3)_3$ | H | $C(CH_3)_3$ | $-O-CH_2-C(O)-$ | $-NO_2$ | 117-119 |
| 26 | H | H | H | $-C(CH_3)_2C_2H_5$ | H | $-O-CH_2-C(O)-$ | $-NO_2$ | |
| 27 | H | $C(CH_3)_3$ | H | $-C(CH_3)_3$ | $CH_3$ | $-O-CH_2-C(O)-$ | $-NO_2$ | |
| 28 | H | $CH(CH_3)C_2H_5$ | H | $-C(CH_3)_3$ | H | $-O-CH_2-C(O)-$ | $-NO_2$ | |
| 29 | H | $CH_3$ | H | $-C(CH_3)_3$ | H | $-S-CH_2-C(O)-$ | $-NO_2$ | 90-91 |
| 30 | H | H | $C(CH_3)_3$ | $-OH$ | $C(CH_3)_3$ | $-O-CH_2-C(O)-$ | $-NO_2$ | |
| 31 | H | H | $C(CH_3)_3$ | H | $C(CH_3)_3$ | $-S-CH_2-CHOH-$ | $-OCOCH_3$ | |
| 32 | H | H | $C(CH_3)_3$ | H | $C(CH_3)_3$ | $-O-CH_2-CHOH-$ | $-H$ | |
| 33 | $CH_3$ | $CH_3$ | $CH_3$ | $-OC(CH_3)_2CH_2CH_2-$ | | $-O-CH_2-CHOH-$ | $-H$ | |

Tabelle - Forts.

| Beispiel Nr. | $R^1$ | $R^2$ | $R^3$ | $R^4$ | $R^5$ | A | $R^6$ | Schmp. (°C) |
|---|---|---|---|---|---|---|---|---|
| 34 | H | H | $C(CH_3)_3$ | H | $C(CH_3)_3$ | $-O-CH_2-COOH-$ | 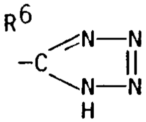 | |
| 35 | H | H | $C(CH_3)_3$ | H | $C(CH_3)_3$ | $-O-CH_2-CHOH-$ | $-CHO$ | |
| 36 | H | H | $C(CH_3)_3$ | H | $C(CH_3)_3$ | $-O-CH_2-CHOH-$ | $-CH_2OH$ | |
| 37 | H | H | $C(CH_3)_3$ | H | $C(CH_3)_3$ | $-O-CH_2-CHOH-$ | 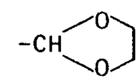 | |
| 38 | H | H | $C(CH_3)_3$ | H | $C(CH_3)_3$ | $-O-CH_2-CHOH-$ | $-SO_3H$ | |
| 39 | H | H | $C(CH_3)_3$ | H | $C(CH_3)_3$ | $-O-CH_2-CHOH-$ | $-SH$ | |
| 40 | H | H | $C(CH_3)_3$ | H | $C(CH_3)_3$ | $-O-CH_2-CHOH-$ | $-COCH_3$ | |
| 41 | H | H | $C(CH_3)_3$ | H | $C(CH_3)_3$ | $-O-CH_2-C(O)-$ | $-C(O)NHOH$ | |

Beispiele für pharmazeutische Zubereitungen:

Beispiel I

Tablette mit 250 mg Wirkstoff
Zusammensetzung für 1000 Tabletten:

| | |
|---|---|
| Wirkstoff des Beispiel Nr. 2: | 250 g |
| Kartoffelstärke: | 100 g |
| Milchzucker: | 50 g |
| Gelatinelösung 4 %: | 45g |
| Talkum: | 10 g |

Herstellung:

Der fein gepulverte Wirkstoff, Kartoffelstärke und Milchzucker werden gemischt. Die Mischung wird mit ca. 45 g 4 %iger wäßriger Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpreßt. Die Tabletten werden in dicht schließende Behälter aus Polypropylen gefüllt.

Beispiel II

Creme aus 0,1 % Wirkstoff

| | |
|---|---|
| Wirkstoff des Beispiels Nr. 10: | 0,1 g |
| Glycerinmonostearat: | 10,0 g |
| Cetylalkohol: | 4,0 g |
| Polyethylenglykol-400-stearat: | 10,0 g |
| Polyethylenglykol-sorbitanmonostearat: | 10,0 g |
| Propylenglykol: | 6,0 g |
| p-Hydroxybenzoesäuremethylester: | 0,2 g |
| Entmineralisiertes Wasser: | ad 100,0 g |

Herstellung:

Der feinst gepulverte Wirkstoff wird mit Propylenglykol suspendiert und die Suspension in die auf 65°C erwärmte Schmelze aus Glycerinmonostearat, Cetylalkohol, Polyethylenglykol-400-stearat und Polyethylenglykolsorbitanmonostearat gerührt. In diese Mischung wird die 70°C heiße Lösung des p-Hydroxybenzoesäuremethylesters in Wasser emulgiert. Nach dem Erkalten wird die Creme über eine Kolloidmühle homogenisiert und in Tuben abgefüllt.

Beispiel III

Puder mit 0,1 % Wirkstoff

| | |
|---|---|
| Wirkstoff des Beispiels Nr. 11: | 0,1 g |
| Zinkoxid: | 10,0 g |
| Magnesiumoxid: | 10,0 g |
| Hochdisperses Siliciumdioxid: | 2,5 g |
| Magnesiumstearat: | 1,0 g |
| Talkum: | 76,4 g |

Herstellung

Der Wirkstoff wird auf einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

## Patentansprüche

1. Oxidierte Diphenylheteroalkane der Formel I

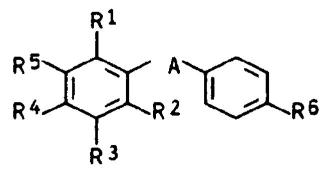

I

in der
A eine der folgenden Gruppen darstellt: $-CHOH-CH_2X-$ oder $-C(O)-CH_2X-$, wobei X mit dem rechten oder dem linken der beiden Phenylringe verbunden sein kann und für ein Sauerstoffatom, eine $-S(O)_n-$ (mit n in der Bedeutung von 0, 1 oder 2) oder eine NH-Gruppe steht,
$R^1$, $R^2$ und $R^3$ unabhängig voneinander ein Wasserstoffatom oder eine $C_1$-$C_4$-Alkylgruppe bedeuten,
$R^4$ und $R^5$ unabhängig voneinander ein Wasserstoffatom, eine $C_1$-$C_5$-Alkylgruppe oder unter Bildung eines Zyklus eine gemeinsame $-C(CH_3)_2-B-C(CH_3)_2-$Gruppe (mit B in der Bedeutung von $-CH_2CH_2-$ oder $-CH(CH_3)-$) oder eine $-OC(CH_3)(Z)CH_2CH_2-$Gruppe (mit Z in der Bedeutung einer $C_1$-$C_2$-Alkylgruppe, die durch den Rest $-OR^9$-Gruppe substituiert sein kann) darstellen, und $R^4$ außerdem eine $OR^9$-Gruppe bedeutet, wobei $R^4$ und $R^5$ gemeinsam einen Zyklus der genannten Art bilden, wenn $R^1$ bis $R^3$ Wasserstoff sind, und $R^4$ und $R^5$ gemeinsam einen Zyklus der genannten Art bilden, oder $R^3$ und $R^5$ jeweils eine verzweigte $C_3$- oder $C_4$-Alkylgruppe darstellen, wenn $R^6$ = H, OH, SH oder $CH_3$,
$R^6$ ein Wasserstoffatom, eine Methyl- oder Nitrilgruppe, den Tetrazolylrest oder die Reste $-CH_2OR^9$, $-OR^{10}$, $-NR^{11}R^{12}$, $-CH_2NR^{11}R^{12}$, $-CH(OR^{13})_2$, $-S(O)_mR^{14}$ (mit m = 1 oder 2), $-SR^{10}$, $-SO_3H$ oder $-COR^{15}$ bedeutet, worin bedeuten:
$R^9$ ein Wasserstoffatom, eine $C_{1-6}$-Alkyl oder eine $C_{1-6}$-Alkanoyl-Gruppe,
$R^{10}$ ein Wasserstoffatom, eine $C_{1-6}$-Alkyl-, eine $C_{1-6}$-Alkanoyl oder Benzoylgruppe oder einen $-CH_2COR^7$-Rest (mit $R^7$ in der Bedeutung von Wasserstoff, einer $C_{1-6}$-Alkyl-, einer $C_{1-6}$-Alkoxy- oder einer Hydroxy-Gruppe),
$R^{11}$, $R^{12}$ unabhängig voneinander ein Wasserstoffatom, eine $C_{1-4}$-Alkyl-, $C_{1-4}$-Alkanoyl oder eine Benzyl- oder Benzoylgruppe,
$R^{13}$ eine $C_{1-6}$-Alkoxygruppe, die gegebenenfalls mit einem zweiten Rest $R^{13}$ unter Einbeziehung der CH-Gruppe ein cyclisches 5- oder 6-gliedriges Acetal bildet,
$R^{14}$ eine $C_{1-6}$-Alkylgruppe,
$R^{15}$ ein Wasserstoffatom, eine Hydroxy-, eine $C_{1-6}$-Alkyl-, eine $C_{1-6}$-Alkoxy-, Phenoxy- oder Benzyloxygruppe, den Rest $-NR^{11}R^{12}$ mit $R^{11}$ und $R^{12}$ in der Bedeutung von Wasserstoff, einer Alkyl- oder gegebenenfalls durch Hydroxy oder $C_{1-4}$-Alkoxy substituierten Benzylgruppe oder den Rest $-NR^{16}OR^{17}$ (mit $R^{16}$, $R^{17}$ = Wasserstoff oder eine $C_1$-$C_3$-Alkylgruppe) darstellen,
sowie deren physiologisch verträgliche Salze.

2. Oxidierte Diphenylheteroalkane der Formel I gemäß Anspruch 1, wobei
X für ein Sauerstoff- oder Schwefelatom oder die NH-Gruppe steht, und
$R^4$ und $R^5$ gemeinsam einen Zyklus der im Anspruch 1 genannten Art oder $R^3$ und $R^5$ jeweils eine verzweigte $C_3$- oder $C_4$-Alkylgruppe darstellen.

3. Oxidierte Diphenylheteroalkane der Formel I gemäß Anspruch 1, wobei $R^6$ einen der Reste $-CH_2OR^9$, $-OR^{10}$, $-SR^{10}$, $-SO_2R^{14}$, $-SO_3H$ oder $-COR^{15}$ bedeutet.

4. Oxidierte Diphenylheteroalkane der Formel I gemäß Anspruch 1, wobei X, $R^4$ und $R^5$ wie im Anspruch 2 definiert sind und $R^6$ für einen der Reste $-CH_2OR^9$, $-OR^{10}$, $-SR^{10}$, $-SO_2R^{14}$, $-SO_3H$ oder $-COR^{15}$ steht.

5. Verfahren zur Herstellung der oxidierten Diphenylheteroalkane der Formel I gemäß einem der Ansprüche 1 bis 4, dadurch gekennzeichnet, daß man
a) - falls A eine $-COCH_2X$-Gruppe bedeutet, bei der X mit dem rechten Phenylring der Formel I verbunden ist - ein Acetophenon der Formel II

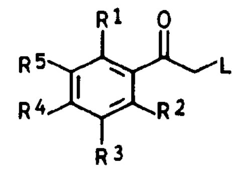

II,

in der $R^1$-$R^5$ die in den Ansprüchen 1 bis 4 angegebenen Bedeutungen haben und L für eine nucleofuge Abgangsgruppe steht, mit einem Benzolderivat der Formel III

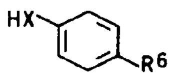

III,

worin $R^6$ die in einem der Ansprüche 1 bis 4 angegebene Bedeutung zukommt und X für ein Sauerstoff- oder Schwefelatom oder die NH-Gruppe steht, in Gegenwart einer Base umsetzt, und, falls X ein Schwefelatom bedeutet, die erhaltenen Thioether gegebenenfalls zu den korrespondierenden Sulfoxiden (X=SO) bzw. Sulfonen ($X=SO_2$) oxidiert,

b) - falls A eine $-XCH_2CO$-Gruppe bedeutet, bei der X mit der linken Phenylgruppe der Formel I verbunden ist - ein Acetophenon der Formel IV

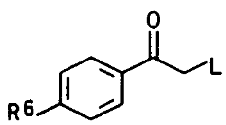

IV,

in der $R^6$ die genannte Bedeutung zukommt und L für eine nucleofuge Abgangsgruppe steht, mit einem Benzolderivat der Formel V

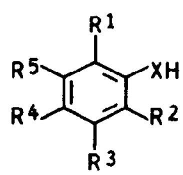

V,

worin $R^1$ bis $R^5$ die genannten Bedeutungen haben und X für ein Sauerstoff- oder Schwefelatom oder die NH-Gruppe steht, in Gegenwart einer Base umsetzt, und falls X ein Schwefelatom bedeutet, die erhaltenen Thioether gegebenenfalls zu den korrespondierenden Sulfoxiden (X=SO) bzw. Sulfonen ($X=SO_2$) oxidiert,

c) - falls A eine $-CH(OH)CH_2X$-Gruppe bedeutet - entweder

c1) Oxirane der Formel VI

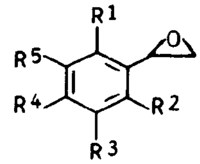

VI

in der $R^1$-$R^5$ die genannten Bedeutungen haben, mit Benzolderivaten der Formel III umsetzt, und, falls X ein Schwefelatom bedeutet, die erhaltenen Thioether gegebenenfalls zu den korrespondierenden Sulfoxiden (X=SO) bzw. Sulfonen ($X=SO_2$) oxidiert, oder

c2) in dem Verfahren a) erhaltenen Ketonen der Formel VII

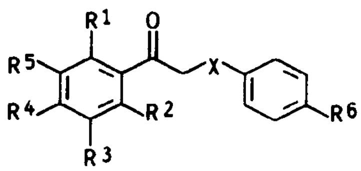

VII,

in der $R^1$-$R^6$ und X die oben angegebenen Bedeutungen haben, nach einer der bekannten Methoden die Ketogruppe reduziert,

d) - falls A eine $-XCH_2CH(OH)$-Gruppe bedeutet - entweder

d1) Oxirane der Formel VIII

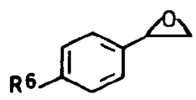

VIII,

in der $R^6$ die genannte Bedeutung hat, mit Benzolderivaten der Formel V umsetzt, und, falls X ein Schwefelatom bedeutet, die erhaltenen Thioether gegebenenfalls zu den korrespondierenden Sulfoxiden (X=SO) bzw. Sulfonen ($X=SO_2$) oxidiert, oder

d2) in den nach Verfahren b) erhaltenen Ketonen der Formel IX

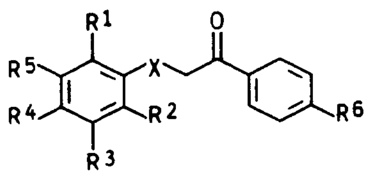

IX,

in der $R^1$ bis $R^6$ und X die genannten Bedeutungen haben, nach einer der bekannten Methoden die Ketogruppe reduziert,

und die so erhaltenen Verbindungen gegebenenfalls nach Standardmethoden in weitere Verbindungen der Formel I und gegebenenfalls ihre physiologisch verträglichen Salze umwandelt.

**6.** Arzneimittel zur topischen Anwendung, das neben üblichen galenischen Hilfsmitteln als Wirkstoff 0,001 bis 1 Gew.% eines oxidierten Diphenylheteroalkans der Formel I gemäß einem der Ansprüche 1 bis 4 enthält.

**7.** Arzneimittel zur systemischen Anwendung, das neben üblichen galenischen Hilfsmitteln als Wirkstoff pro Einzeldosis 0,1 bis 250 mg eines oxidierten Diphenylheteroalkans der Formel I gemäß einem der Ansprüche 1 bis 4 enthält.

**8.** Kosmetische Zubereitung, die neben üblichen kosmetischen Hilfsmitteln 0,001 bis 1 Gew.% eines oxidierten Diphenylheteroalkans der Formel I gemäß einem der Ansprüche 1 bis 4 enthält.

**9.** Arzneimittel nach Anspruch 6 zur Behandlung von Akne oder Psoriasis oder anderen mit pathologisch veränderter Verhornung einhergehenden dermatologischen Erkrankungen sowie von Ekzemen, Warzen und Vitiligo.

**10.** Arzneimittel nach Anspruch 6 zur Behandlung von durch UV-Licht oder iatrogen bedingten Schädigungen der Haut.

**11.** Arzneimittel nach Anspruch 6 oder 7 zur Behandlung von Präkanzerosen und Tumoren.

**12.** Arzneimittel nach Anspruch 6 oder 7 zur Behandlung von rheumatischen und arthritischen Erkrankungen.

**13.** Arzneimittel nach Anspruch 6 zur Behandlung von trockenen Augen und anderen Corneopathien.

## Claims

1. An oxidized diphenylheteroalkane of the formula I

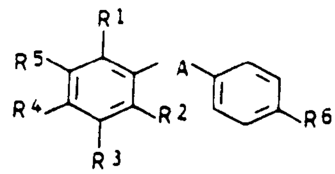

I

where
A is one of the following: -CHOH-$CH_2X$- or -C(O) -$CH_2X$-, where X can be bonded to the right or left of the two phenyl rings and is oxygen, -$S(O)_n$- ( with n being 0, 1 or 2) or NH,
$R^1$, $R^2$ and $R^3$ are, independently of one another, hydrogen or $C_1$-$C_4$-alkyl,
$R^4$ and $R^5$ are, independently of one another, hydrogen, $C_1$-$C_5$-alkyl or together, with the formation of a ring, -$C(CH_3)_2$-B-$C(CH_3)_2$- (with B being -$CH_2CH_2$- or -$CH(CH_3)$-) or -$OC(CH_3)$ (Z) $CH_2CH_2$- (with Z being $C_1$-$C_2$-alkyl which can be substituted by -$OR^9$), and $R^4$ is additionally -$OR^9$, where $R^4$ and $R^5$ together form a ring of the said type when $R^1$ to $R^3$ are hydrogen, and $R^4$ and $R^5$ together form a ring of the said type, or $R^3$ and $R^5$ are each branched $C_3$- or $C_4$-alkyl when $R^6$ = H, OH, SH or $CH_3$,
$R^6$ is hydrogen, methyl, cyano, tetrazolyl or -$CH_2OR^9$, -$OR^{10}$, -$NR^{11}R^{12}$, -$CH_2NR^{11}R^{12}$, -$CH(OR^{13})_2$, -$S(O)_mR^{14}$ (with m = 1 or 2 ), -$SR^{10}$, -$SO_3H$ or -$COR^{15}$, where
$R^9$ is hydrogen, $C_{1}$-$_{6}$-alkyl or $C_{1}$-$_{6}$-alkanoyl,
$R^{10}$ is hydrogen, $C_{1-6}$-alkyl, $C_{1-6}$-alkanoyl or benzoyl or -$CH_2COR^7$ (with $R^7$ being hydrogen, $C_{1-6}$-alkyl, $C_{1-6}$-alkoxy or hydroxyl ),
$R^{11}$ and $R^{12}$ are, independently of one another, hydrogen, $C_{1-4}$-alkyl, $C_{1-4}$-alkanoyl or benzyl or benzoyl,
$R^{13}$ is $C_{1-6}$-alkoxy, it being possible for two $R^{13}$ radicals to form together with the CH a cyclic 5- or 6-membered acetal,
$R^{14}$ is $C_{1-6}$-alkyl,
$R^{15}$ is hydrogen, hydroxyl, $C_{1-6}$-alkyl, $C_{1-6}$-alkoxy, phenoxy or benzyloxy, -$NR^{11}R^{12}$ with $R^{11}$ and $R^{12}$ being hydrogen, alkyl, or benzyl which can be substituted by hydroxyl or $C_{1-4}$-alkoxy, or -$NR^{16}OR^{17}$ (with $R^{16}$ and $R^{17}$ = hydrogen or $C_1$-$C_3$-alkyl ),
and the physiologically tolerated salts thereof.

2. An oxidized diphenylheteroalkane of the formula I as claimed in claim 1, where
X is oxygen, sulfur or NH, and
$R^4$ and $R^5$ together are a ring of the type mentioned in claim 1, or $R^3$ and $R^5$ are each a branched $C_3$- or $C_4$-alkyl.

3. An oxidized diphenylheteroalkane of the formula I as claimed in claim 1, where $R^6$ is -$CH_2OR^9$, -$OR^{10}$, -$SR^{10}$, -$SO_2R^{14}$, -$SO_3H$ or -$COR^{15}$.

4. An oxidized diphenylheteroalkane of the formula I as claimed in claim 1, where X, $R^4$ and $R^5$ are as defined in claim 2, and $R^6$ is -$CH_2OR^9$, -$OR^{10}$, -$SR^{10}$, -$SO_2R^{14}$, -$SO_3H$ or -$COR^{15}$.

5. A process for the preparation of an oxidized diphenylheteroalkane of the formula I as claimed in any one of claims 1 to 4, which comprises
a) if A is -$COCH_2X$- where X is connected to the phenyl on the right in formula I, reacting an acetophenone of the formula II

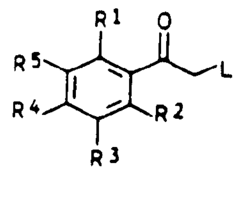

II,

where $R^1$-$R^5$ have the meanings specified in claims 1 to 4, and L is a nucleofugic leaving group, with a benzene derivative of the formula III

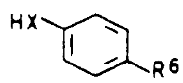

III,

where $R^6$ has the meaning specified in any one of claims 1 to 4, and X is oxygen, sulfur or NH, in the presence of a base and, if X is sulfur, possibly oxidizing the resulting thioethers to the corresponding sulfoxides (X=SO) or sulfones ($X=SO_2$),

b) if A is $-XCH_2CO-$ where X is connected to the phenyl on the left in formula I, reacting an acetophenone of the formula IV

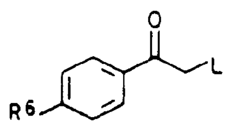

IV,

where $R^6$ has the said meaning, and L is a nucleofugic leaving group, with a benzene derivative of the formula V

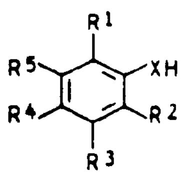

V,

where $R^1$ to $R^5$ have the abovementioned meanings, and X is oxygen, sulfur or NH, in the presence of a base, and, if X is sulfur, possibly oxidizing the resulting thioethers to the corresponding sulfoxides (X=SO) or sulfones ($X=SO_2$),

c) if A is $-CH(OH)CH_2X-$, either

c1) reacting an oxirane of the formula VI

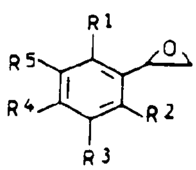

VI

where $R^1$-$R^5$ have the abovementioned meanings, with a benzene derivative of the formula III and, if X is sulfur, possibly oxidizing the resulting thioethers to the corresponding sulfoxides (X=SO) or sulfones ($X=SO_2$), or

c2) reducing the keto group in a ketone of the formula VII obtained in process a)

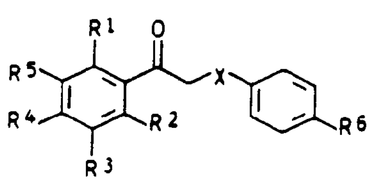

VII,

where $R^1$-$R^6$ and X have the abovementioned meanings, by a conventional method,

d) if A is $-XCH_2CH(OH)-$, either

d1) reacting an oxirane of the formula VIII

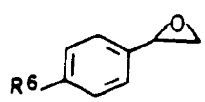

VIII,

where $R^6$ has the abovementioned meaning, with a benzene derivative of the formula V and, if X is sulfur, possibly oxidizing the resulting thioethers to the corresponding sulfoxides (X=SO) or sulfones ($X=SO_2$), or

d2) reducing the keto group in a ketone of the formula IX obtained in process b)

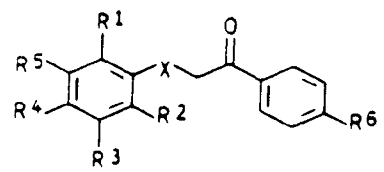

IX,

where $R^1$-$R^6$ and X have the abovementioned meanings, by a conventional method,

and possibly converting the resulting compounds by standard methods into other compounds of the formula I or the physiologically tolerated salts thereof.

**6.** A drug for topical use which contains as active substance 0.001 to 1 % by weight of an oxidized diphenylheteroalkane of the formula I as claimed in any one of claims 1 to 4 in addition to conventional pharmaceutical auxiliaries.

**7.** A drug for systemic use which contains as active substance 0.1 to 250 mg of an oxidized diphenylheteroalkane of the formula I as claimed in any one of claims 1 to 4 per single dose, in addition to conventional pharmaceutical auxiliaries.

**8.** A cosmetic preparation which contains 0.001 to 1 % by weight of an oxidized diphenylheteroalkane of the formula I as claimed in any one of claims 1 to 4 in addition to conventional cosmetic auxiliaries.

**9.** A drug as claimed in claim 6 for the treatment of acne or psoriasis or other dermatological disorders associated with pathological keratinization, and of eczema, warts and vitiligo.

**10.** A drug as claimed in claim 6 for the treatment of UV-induced or iatrogenic damage to the skin.

**11.** A drug as claimed in claim 6 or 7 for the treatment of precanceroses and tumors.

**12.** A drug as claimed in claim 6 or 7 for the treatment of rheumatic and arthritic disorders.

**13.** A drug as claimed in claim 6 for the treatment of dry eyes and other corneopathies.

## Revendications

**1.** Diphénylhétéroalcanes oxydés de formule I

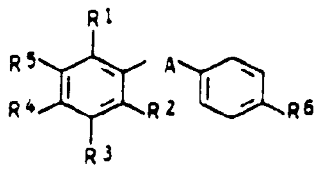

I

dans laquelle

A représente l'un des groupes suivants : $-CHOH-CH_2X-$ ou $-C(O)-CH_2X-$ dans lesquels X, pouvant être relié au cycle phényle de droite ou au cycle phényle de gauche, représente un atome d'oxygène, un groupe $-S(O)_n-$(avec n = 0, 1 ou 2) ou un groupe -NH-,

$R^1$, $R^2$ et $R^3$ représentent chacun, indépendamment les uns des autres, un atome d'hydrogène ou un groupe alkyle en C 1-C 4,

$R^4$ et $R^5$ représentent chacun, indépendamment l'un de l'autre, un atome d'hydrogène, un groupe alkyle en C 1-C 5 ou, ensemble, avec formation d'un cycle, un groupe $-C(CH_3)_2-B-C(CH_3)_2-$(dans lequel B représente $-CH_2CH_2-$ ou $-CH(CH_3)-$) ou un groupe $-OC(CH_3)(Z)CH_2CH_2-$(Z représentant un groupe alkyle en C 1-C 2 qui peut être substitué par le groupe $-OR^9$), et $R^4$ peut en outre représenter un groupe $-OR^9$, $R^4$ et $R^5$ forment ensemble un cycle du type mentionné lorsque $R^1$ à $R^3$ représentent l'hydrogène, et $R^4$ et $R^5$ formant ensemble un cycle du type mentionné, ou bien $R^3$ et $R^5$ représentent chacun un groupe alkyle ramifié en C 3 ou C 4 lorsque $R^6$ = H, OH, SH ou $CH_3$,

$R^6$ représente un atome d'hydrogène, un groupe méthyle ou nitrile, le radical tétrazolyle ou les groupes $-CH_2OR^9$, $-OR^{10}$, $-NR^{11}R^{12}$, $-CH_2NR^{11}R^{12}$, $-CH(OR^{13})_2$, $-S(O)_mR^{14}$ (avec m = 1 ou 2), $-SR^{10}$, $-SO_3H$ ou $-COR^{15}$, dans lesquels :

$R^9$ représente un atome d'hydrogène, un groupe alkyle en C 1-C 6 ou alcanoyle en C 1-C 6,

$R^{10}$ représente un atome d'hydrogène, un groupe alkyle en C 1-C 6, alcanoyle en C 1-C 6 ou benzoyle, ou un groupe $-CH_2COR^7$ (dans lequel $R^7$ représente l'hydrogène, un groupe alkyle en C 1-C 6, alcoxy en C 1-C 6 ou hydroxy),

$R^{11}$, $R^{12}$ représentent chacun, indépendamment l'un de l'autre, un atome d'hydrogène, un groupe alkyle en C 1-C 4, alcanoyle en C 1-C 4 ou benzyle ou benzoyle,

$R^{13}$ représente un groupe alcoxy en C 1-C 6 qui le cas échéant forme avec un deuxième groupe $R^{13}$, et avec inclusion du groupe CH, un acétal cyclique à 5 ou 6 chaînons,

$R^{14}$ représente un groupe alkyle en C1-C 6,

$R^{15}$ représente un atome d'hydrogène, un groupe hydroxy, alkyle en C1-C 6, alcoxy en C 1-C 6, phénoxy ou benzyloxy, le groupe $-NR^{11}R^{12}$ dans lequel $R^{11}$ et $R^{12}$ représentent l'hydrogène, un groupe alkyle ou un groupe benzyle éventuellement substitué par un groupe hydroxy ou alcoxy en C 1-C 4, ou le groupe $-NR^{16}OR^{17}$ (dans lequel $R^{16}$, $R^{17}$ = hydrogène ou groupe alkyle en C1-C 3),

et leurs sels acceptables pour l'usage pharmaceutique.

2. Diphénylhétéroalcanes oxydés de formule I selon la revendication 1, dans laquelle
X représente un atome d'oxygène ou de soufre ou le groupe NH, et
$R^4$ et $R^5$ forment ensemble un cycle du type décrit dans la revendication 1, ou bien
$R^3$ et $R^5$ représentent chacun un groupe alkyle ramifié en C 3-C 4.

3. Diphénylhétéroalcanes oxydés de formule I selon la revendication 1, dans laquelle $R^6$ représente l'un des groupes $-CH_2OR^9$, $-OR^{10}$, $-SR^{10}-SO_2R^{14}$, $-SO_3H$ ou $-COR^{15}$.

4. Diphénylhétéroalcanes oxydés de formule I de la revendication 1, dans laquelle X, $R^4$ et $R^5$ ont les significations indiquées dans la revendication 2 et $R^6$ représente l'un des groupes $-CH_2OR^9$, $-OR^{10}$, $-SR^{10}$, $-SO_2R^{14}$, $-SO_3H$ ou $-COR^{15}$.

5. Procédé de préparation des diphénylhétéroalcanes oxydés de formule I selon l'une des revendications 1 à 4, caractérisé en ce que :

a) - lorsque A représente un groupe $-COCH_2X-$ dans lequel X est relié au cycle phényle de droite de la formule I - on fait réagir en présence d'une base une acétophénone de formule II

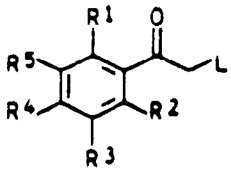

II,

dans laquelle $R^1$ à $R^5$ ont les significations indiquées dans les revendications 1 à 4 et L représente un groupe éliminable nucléofuge, avec un dérivé benzénique de formule III

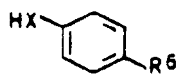

III,

dans laquelle $R^6$ a les significations indiquées dans l'une des revendications 1 à 4, et X représente un atome d'oxygéne ou de soufre ou le groupe -NH-, et lorsque X représente un atome de soufre, on oxyde éventuellement les thioéthers obtenus en les sulfoxydes correspondants (X = SO) ou les sulfones correspondantes (X = $SO_2$);

b) - lorsque A représente un groupe -$XCH_2CO$- dans lequel X est relié au groupe phényle de gauche de la formule I - on fait réagir en présence d'une base une acétophénone de formule IV

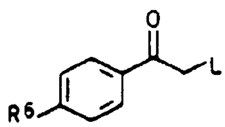

IV,

dans laquelle $R^6$ a les significations indiquées ci-dessus et L représente un groupe éliminable nucléofuge, avec un dérivé benzénique de formule V

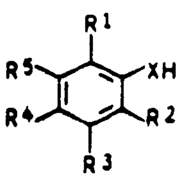

V,

dans laquelle $R^1$ à $R^5$ ont les significations indiquées ci-dessus et X représente un atome d'oxygène ou de soufre ou le groupe -NH- et, lorsque X représente un atome de soufre, on oxyde éventuellement les thioéthers obtenus en les sulfoxydes correspondants (X = SO) ou les sulfones correspondantes (X = $SO_2$),

c) - lorsque A représente un groupe -$CH(OH)CH_2X$- -,

c1) on fait réagir des oxirannes de formule VI

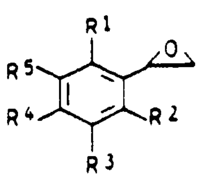

VI

dans laquelle $R^1$ à $R^5$ ont les significations indiquées ci-dessus, avec des dérivés benzéniques de formule III et, lorsque X représente un atome de soufre, on oxyde éventuellement les thioéthers

obtenus en les sulfoxydes correspondants (X = SO) ou en les sulfones correspondants (X = $SO_2$), ou bien

c2) dans les cétones de formule VII

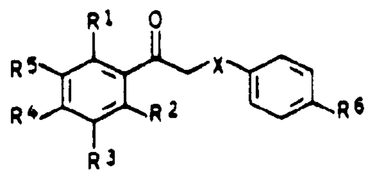

VII,

dans laquelle $R^1$ à $R^6$ et X ont les significations indiquées ci-dessus, obtenues par le procédé a), on réduit le groupe céto par l'un des procédés connus à cet effet,

d) - lorsque A représente un groupe $-XCH_2CH(OH)-$ -,

d1) on fait réagir des oxirannes de formule VIII

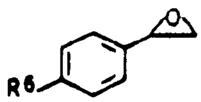

VIII,

dans laquelle $R^6$ a les significations indiquées ci-dessus, avec des dérivés benzéniques de formule V et, lorsque X représente un atome de soufre, on oxyde éventuellement les thioéthers obtenus en les sulfoxydes correspondants (X = SO) ou les sulfones correspondantes (X = $SO_2$), ou bien

d2) dans les cétones de formule IX

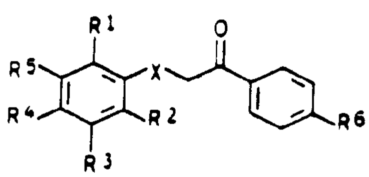

IX,

dans laquelle $R^1$ à $R^6$ et X ont les significations indiquées ci-dessus, obtenues par le procédé b), on réduit le groupe céto par l'un des procédés connus à cet effet, et le cas échéant, on convertit les composés ainsi obtenus par des procédés classiques en d'autres composés de formule I ou en leurs sels acceptables pour l'usage pharmaceutique.

6. Médicament pour l'application topique contenant, avec des produits auxiliaires galéniques usuels, de 0,001 à 1 % en poids d'un diphénylhétéroalcane oxydé de formule I selon l'une des revendications 1 à 4 en tant que substance active.

7. Médicament pour l'administration systèmique contenant, avec des produits auxiliaires galéniques usuels, et par unité de dosage, de 0,1 à 250 mg d'un diphénylhétéroalcane oxydé de formule I selon l'une des revendications 1 à 4 en tant que substance active.

8. Composition cosmétique contenant, avec des produits auxiliaires cosmétiques usuels, de 0,001 à 1 % en poids d'un diphénylhétéroalcane oxydé de formule I selon l'une des revendications 1 à 4.

9. Médicament selon la revendication 6, pour le traitement de l'acné ou du psoriasis ou d'autres maladies dermatologiques s'accompagnant d'une kératinisation pathologique, et pour le traitement des eczémas, des verrues et du vitiligo.

10. Médicament selon la revendication 6, pour le traitement des affections de la peau provoquées par la lumière UV ou d'origine iatrogène.

11. Médicament selon la revendication 6 et 7, pour le traitement des précancéroses et des tumeurs.

12. Médicament selon la revendication 6 ou 7, pour le traitement des maladies rhumatismales et arthritiques.

13. Médicament selon la revendication 6, pour le traitement des yeux secs et d'autres cornéopathies.